(19) Europäisches Patentamt
European Patent Office
Office européen des brevets

(11) Veröffentlichungsnummer: **0 084 020**
**B1**

(12) **EUROPÄISCHE PATENTSCHRIFT**

(45) Veröffentlichungstag der Patentschrift: **16.09.87**

(51) Int. Cl.⁴: **C 07 D 239/46**, C 07 D 239/52, C 07 D 239/48, A 01 N 47/36

(21) Anmeldenummer: **83810005.5**

(22) Anmeldetag: **05.01.83**

(54) **N-Arylsulfonyl-N'-pyrimidinylharnstoffe.**

(30) Priorität: **11.01.82 CH 124/82**
**02.09.82 CH 5224/82**

(43) Veröffentlichungstag der Anmeldung:
**20.07.83 Patentblatt 83/29**

(45) Bekanntmachung des Hinweises auf die Patenterteilung:
**16.09.87 Patentblatt 87/38**

(84) Benannte Vertragsstaaten:
**AT BE CH DE FR GB IT LI NL SE**

(56) Entgegenhaltungen:
**EP - A - 0 009 419**
**US - A - 4 169 719**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Meyer, Willy, Talweg 49, CH-4125 Riehen (CH)**
Erfinder: **Gass, Karl, Blumenrain 4, CH-4312 Magden (CH)**
Erfinder: **Töpfl, Werner, Dr., Dorneckstrasse 68, CH-4143 Dornach (CH)**
Erfinder: **Schurter, Rolf, Dr., Holzmattstrasse 45, CH-4102 Binningen (CH)**
Erfinder: **Pissiotas, Georg, Dr., Breslauerstrasse 8, D-7850 Lörrach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 084 020 B1

**Beschreibung**

Die vorliegende Erfindung betrifft neue, herbizid wirksame und pflanzenwuchsregulierende N-Arylsulfonyl-N′-pyrimidinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Aminopyrimidine.

Die erfindungsgemässen N-Arylsulfonyl-N′-pyrimidinylharnstoffe entsprechen der allgemeinen Formel I

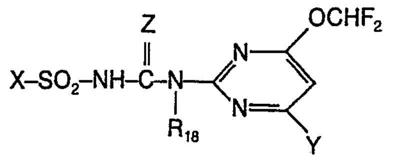

(I)

worin

X einen Rest der Formel

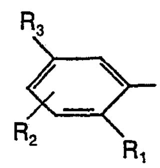

oder

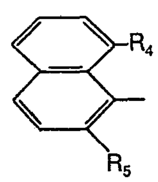

,

Y $C_1$–$C_3$-Alkyl, $C_1$–$C_3$-Halogenalkyl, $C_1$–$C_3$-Alkoxy, $C_1$–$C_3$-Halogenalkoxy, $C_2$–$C_3$-Alkoxyalkyl, $C_1$–$C_3$-Alkylthio, Halogen oder $-NR_{16}R_{17}$,

Z Sauerstoff oder Schwefel,

$R_1$ Wasserstoff, Halogen, Cyan, Nitro, $C_1$–$C_4$-Halogenalkyl, $C_1$–$C_4$-Alkyl, $C_1$–$C_4$-Alkoxy, $-CO-R_6$, $-NR_7R_8$, $-S(O)_m-C_1$–$C_4$-Alkyl oder $-SO_2R_9$,

$R_2$ Wasserstoff, Fluor, Chlor, Brom, Nitro, Trifluormethyl, $-NR_{20}R_{21}$, Methyl, Äthyl, Methoxy, Äthoxy oder $-S(O)_m-C_1$–$C_4$-Alkyl,

$R_3$ Wasserstoff, Fluor, Chlor, Brom, Amino, Nitro oder Methoxy,

$R_6$ Wasserstoff, $C_1$–$C_4$-Alkyl, $C_3$–$C_5$-Alkenyloxy, $C_3$–$C_5$-Alkinyloxy, $C_1$–$C_4$-Halogenalkyl, $C_1$–$C_5$-Alkylthio, Phenoxy, Benzyloxy, $-NR_{10}R_{11}$ oder gegebenenfalls durch 1–3 Halogenatome oder $C_1$–$C_3$-Alkoxy substituiertes $C_1$–$C_5$-Alkoxy,

$R_7$ Wasserstoff, Methoxy, Äthoxy, $C_1$–$C_4$-Alkyl oder $-CO-R_{12}$,

$R_8$ Wasserstoff oder $C_1$–$C_4$-Alkyl,

$R_9$ eine Gruppe $-O-R_{13}$ oder $-NR_{14}R_{15}$,

$R_{13}$ gegebenenfalls durch 1–3 Halogenatome substituiertes $C_1$–$C_4$-Alkyl, Phenyl oder Benzyl,

$R_{18}$ Wasserstoff, $C_1$–$C_3$-Alkyl oder $C_1$–$C_3$-Alkoxy und

m die Zahlen Null, eins oder zwei bedeuten, wobei $R_4$ die gleiche Bedeutung wie $R_2$; $R_5$ die gleiche Bedeutung wie $R_1$; $R_{10}$, $R_{11}$, $R_{14}$ und $R_{20}$ die gleiche Bedeutung wie $R_7$; und $R_{12}$, $R_{15}$, $R_{16}$, $R_{17}$ und $R_{21}$ die gleiche Bedeutung wie $R_8$ haben, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 9419 oder 30 141 beschrieben.

In den Definitionen ist unter Alkyl geradkettiges oder verzweigtes Alkyl zu verstehen; z.B.: Methyl, Äthyl, n-Propyl, i-Propyl oder die vier isomeren Butyl.

Unter Alkoxy ist zu verstehen: Methoxy, Äthoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Äthoxy oder i-Propyloxy.

Beispiele für Alkylthio sind Methylthio, Äthylthio, n-Propylthio, i-Propylthio, n-Butylthio oder n-Pentylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl oder n-Propylsulfonyl, insbesondere aber Methylsulfonyl und Äthylsulfonyl.

Unter Halogen in den Definitionen sowie als Teil in Halogenalkoxy sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl sowie isomere Pentinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den Verbindungen der Formel I sind als bevorzugt solche Verbindungen anzusehen, die der engeren Unterformel Ia

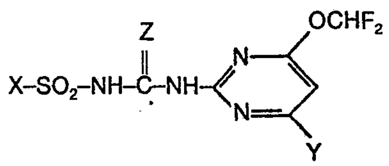 (Ia)

entsprechen, worin

X einen Rest der Formel

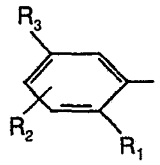 oder 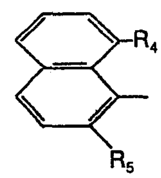,

Y Methyl, Äthyl, Methoxy, Äthoxy, Difluormethoxy, Dimethylamino oder Äthylmethylamino,

Z Sauerstoff oder Schwefel,

$R_1$ Wasserstoff, Fluor, Chlor, Brom, Jod, Cyan, Nitro, Trifluormethyl, $C_1$–$C_4$-Alkyl, $C_1$–$C_4$-Alkoxy, $-CO-R_6$, $-NR_7R_8$, $-S(O)_m-C_1-C_4$-Alkyl oder $-SO_2-R_9$,

$R_2$ Wasserstoff, Fluor, Chlor, Brom, Nitro, Trifluormethyl, Amino, Methyl, Äthyl, Methoxy, Äthoxy oder $-S(O)_m-C_1-C_3$-Alkyl,

$R_3$ Wasserstoff, Fluor, Chlor, Brom, Nitro oder Methoxy,

$R_6$ Wasserstoff, $C_1$–$C_3$-Alkyl, $C_3$–$C_5$-Alkenyloxy, $C_3$–$C_5$-Alkinyloxy, $C_1$–$C_5$-Alkylthio, Phenoxy, Benzyloxy, $-NR_{10}R_{11}$ oder gegebenenfalls durch 1–3 Halogenatome substituiertes $C_1$–$C_5$-Alkoxy,

$R_7$ Wasserstoff, Methoxy, Äthoxy, $C_1$–$C_4$-Alkyl oder $-CO-R_{12}$,

$R_8$ Wasserstoff oder $C_1$–$C_4$-Alkyl,

$R_9$ eine Gruppe $-O-R_{13}$ oder $-NR_{14}R_{15}$,

$R_{13}$ $C_1$–$C_4$-Alkyl, Phenyl oder Benzyl und

m die Zahlen Null, eins oder zwei bedeuten, wobei $R_4$ die gleiche Bedeutung wie $R_2$; $R_5$ die gleiche Bedeutung wie $R_1$; $R_{10}$ und $R_{14}$ die gleiche Bedeutung wie $R_7$ und $R_{11}$, $R_{12}$ und $R_{15}$ die gleiche Bedeutung wie $R_8$ haben, sowie den Salzen dieser Verbindungen.

Von den erfindungsgemässen Verbindungen der Formel I und Ia sind die Verbindungen bevorzugt, in denen

a) X den gegebenenfalls substituierten Phenylrest,
b) Y einen Rest mit höchstens 2 Kohlenstoffatomen,
c) Z Sauerstoff,
d) $R_3$ und $R_4$ Wasserstoff und
e) $R_{18}$ Wasserstoff oder Methyl bedeuten.

Durch Kombination einiger bevorzugter Merkmale ergeben sich folgende weiter bevorzugte Gruppen von Verbindungen der Formel I und Ia, in denen

X den gegebenenfalls substituierten Phenylrest,

Y einen Rest mit höchstens 2 Kohlenstoffatomen,

Z Sauerstoff und $R_3$ und $R_{18}$ Wasserstoff bedeuten.

Innerhalb dieser Gruppe sind weiterhin die Verbindungen bevorzugt, in denen $R_2$ für Wasserstoff steht.

Eine besonders hervorzuhebende Untergruppe der Verbindungen der Formel I zeichnen sich dadurch aus, dass X den gegebenenfalls substituierten Phenylkern, Y einen Rest mit höchstens 2 Kohlenstoffatomen, Z Sauerstoff und $R_{18}$ Wasserstoff bedeuten, wobei $R_1$ für Wasserstoff, Halogen, Nitro, $C_1$–$C_4$-Halogenalkyl, $C_1$–$C_4$-Alkoxy, $C_1$–$C_4$-Alkyl, $-S(O)_m-C_1-C_4$-Alkyl, $-SO_2-N(CH_3)_2$, $-SO_2-OCH_2CF_3$ oder $-CO-R_6$, $R_2$ für Wasserstoff, Fluor, Chlor, Nitro, Amino, Trifluormethyl, Methyl, Methoxy, Äthoxy oder $-S(O)_m-C_1-C_4$-Alkyl, $R_6$ für Wasserstoff, Methyl, $C_3$–$C_5$-Alkenyloxy, $C_3$–$C_5$-Alkinyloxy, $C_1$–$C_3$-Alkylthio, Dimethylamino, Methylamino, Amino oder gegebenenfalls durch 1 bis 3 Halogenatome oder $C_1$–$C_3$-Alkoxy substituiertes $C_1$–$C_5$-Alkoxy und m für die Zahlen Null, eins oder zwei stehen.

Als bevorzugte Einzelverbindungen sind zu nennen:

N-(2-Chlorphenyl-sulfonyl)-N'-(4-difluormethoxy-6-methyl-pyrimidin-2-yl)-harnstoff,

N-(2-Methoxycarbonylphenyl-sulfonyl)-N'-(4-difluormethoxy-6-methoxy-pyrimidin-2-yl)-harnstoff,

N-(2-Nitrophenyl-sulfonyl)-N'-(4-difluormethoxy-6-methyl-pyrimidin-2-yl)-harnstoff,

N-(2-Methoxycarbonylphenyl-sulfonyl)-N'-[4,6-bis-(difluormethoxy)-pyrimidin-2-yl]-harnstoff und

N-(2-Methoxycarbonylphenyl-sulfonyl)-N'-(4-difluormethoxy-6-methyl-pyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Arylsulfonamid der Formel II

$$X-SO_2-NH_2 \qquad (II),$$

worin X die unter Formel I gegebene Bedeutung hat, in Gegenwart einer Base mit einem N-Pyrimidinylcarbamat der Formel III

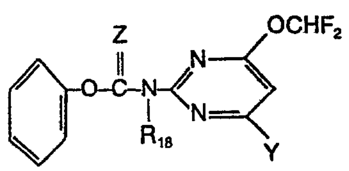 (III),

worin $R_{18}$, Y und Z die unter Formel I gegebene Bedeutung haben, umsetzt.

Nach einem zweiten Verfahren gelangt man zu Verbindungen der Formel I, indem man ein Arylsulfonylisocyanat oder -isothiocyanat der Formel IV

$X\text{–}SO_2\text{–}N=C=Z$ (IV),

worin X und Z die unter Formel I gegebene Bedeutung haben, gegebenenfalls in Gegenwart einer Base, mit einem Aminopyrimidin der Formel V

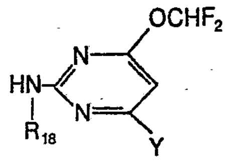

(V),

worin $R_{18}$ und Y die unter Formel I gegebene Bedeutung haben, umsetzt.

Nach einem weiteren Verfahren werden die Verbindungen der Formel I, in denen $R_{18}$ Wasserstoff bedeutet, hergestellt, indem man ein Arylsulfonamid der oben angegebenen Formel II gegebenenfalls in Gegenwart einer Base mit einem Isocyanat oder Isothiocyanat der Formel VI

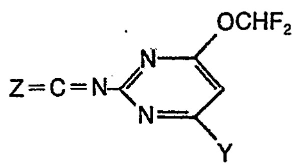

(VI),

worin Y und Z die unter Formel I gegebene Bedeutung haben, umsetzt.

Schliesslich kann man die Verbindungen der Formel I auch erhalten, indem man ein N-Arylsulfonylcarbamat der Formel VII

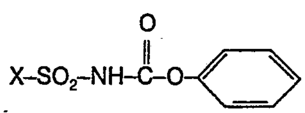

(VII),

worin X die unter Formel I gegebene Bedeutung hat, mit einem Aminopyrimidin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Diese Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen $-20°$ und $+120\,°C$. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Als Basen können sowohl organische Basen wie Amine, wie Triäthylamin, Chinuclidin, Pyridin etc. als auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahmen.

Die Zwischenprodukte der Formeln II, IV und VII sind bekannt oder können analog zu bekannten Verfahren hergestellt werden.

Die Zwischenprodukte der Formel V sind teilweise in der europäischen Patentanmeldung Nr. 82810300.2 beschrieben. Die neuen Ausgangsverbindungen dieses Typs entsprechen der allgemeinen Formel Va

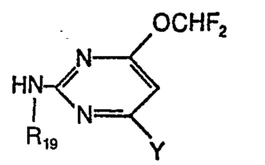

(Va)

worin Y die unter Formel I gegebene Bedeutung hat und $R_{19}$ für $C_1$–$C_3$-Alkyl oder $C_1$–$C_3$-Alkoxy steht. Diese neuen Zwischenprodukte wurden speziell für die Synthese der Wirkstoffe der Formel I entwickelt und bilden daher einen weiteren Gegenstand der vorliegenden Erfindung.

Die Verbindungen der Formel Va werden erhalten, indem man ein Aminopyrimidin der Formel VIII

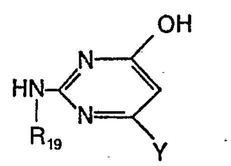

(VIII)

worin Y die unter Formel I gegebene Bedeutung hat und $R_{19}$ für $C_1$–$C_5$-Alkyl oder $C_1$–$C_3$-Alkoxy steht, in Gegenwart einer Base mit Difluor-chlormethan oder Difluor-brommethan umsetzt.

Das Verfahren zur Herstellung der Verbindungen der Formel Va wird mit Vorteil in einem inerten polaren Lösungsmittel oder Lösungsmittelgemisch ausgeführt. Geeignete Lösungsmittel sind Äther wie Dioxan, Tetrahydrofuran, Äthylenglykoldimethyläther, Diäthylenglykoldimethyläther; Alkohole wie Methanol, Äthanol; Ketone wie Aceton, Äthylmethylketon; Dimethylformamid; Acetonitril oder Dimethylsulfoxid. Als Basen sind in besonderer Weise geeignet; Natrium- und Calciumhydrid, Kalium- und Natriumhydroxid, Kalium- und Natriumcarbonat. In geeigneten Fällen kann die Base als wässrige Lösung zugesetzt werden.

Die Ausgangsmaterialien der Formel VIII sind bekannt oder werden analog zu bekannten Verfahren erhalten.

Die Zwischenprodukte der Formeln III und VI sind zum Teil aus der europäischen Patentanmeldung Nr. 82810300.2 bekannt oder werden analog zu bekannten Verfahren aus den Zwischenprodukten der Formel V hergestellt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Stoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei – im Vergleich zu anderen Herbiziden und Wuchsregulatoren – sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüber hinaus eine konzentrationsabhängige pflanzenwuchshemmende Wirkung. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die «cover crops» jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z.B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodendeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykohl, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie

epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979.

Sisley and Wood, «Encyclopedia of Surface Active Agents».

Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

| | | |
|---|---|---|
| Emulgierbare Konzentrate | | |
| Aktiver Wirkstoff: | 1 bis 20%, bevorzugt | 5 bis 10% |
| oberflächenaktives Mittel: | 5 bis 30%, vorzugsweise | 10 bis 20% |
| flüssiges Trägermittel: | 50 bis 94%, vorzugsweise | 70 bis 85%. |

| | |
|---|---|
| Stäube | |
| Aktiver Wirkstoff: | 0,1 bis 10%, vorzugsweise 0,1 bis 1% |
| festes Trägermittel: | 99,9 bis 90%, vorzugsweise 99,9 bis 99%. |
| Suspension-Konzentrate | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |
| Benetzbare Pulver | |
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |
| Granulate | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel 1:
N-(2-Chlorphenyl-sulfonyl)-N'-(4-difluormethoxy-6-methyl-pyrimidin-2-yl)-harnstoff

b) Eine Lösung von 4,9 g (0,0225 Mol) 2-Chlorphenyl-sulfonyl-isocyanat und 3,5 g (0,02 Mol) 2-Amino-4-difluormethoxy-6-methyl-pyrimidin in 70 ml Dioxan wird für 2 Stunden bei 60–70 °C gerührt. Durch Filtration, Eindampfen und Versetzen des Rückstandes mit Äther erhält man 3,8 g (49,9% d. Th.)
N-(2-Chlorphenyl-sulfonyl)-N'-(4-difluormethoxy-6-methyl-pyrimidin-2-yl)-harnstoff,
Smp. 169–70 °C.

Beispiel 2:
N-(2-Methoxycarbonylphenyl-sulfonyl)-N'-[4,6-bis-(difluormethoxy)-pyrimidin-2-yl]-harnstoff

Analog zu Beispiel 1b erhält man aus 3,62 g (0,0158 Mol) 2-Methoxycarbonylphenyl-sulfonyl-isocyanat und 2,63 g (0,0116 Mol) 2-Amino-4,6-bis-(difluormethoxy)-pyrimidin in 50 ml Dioxan 3,9 g (73,7% d. Th.)
N-(2-Methoxycarbonylphenyl-sulfonyl)-N'-[4,6-bis-(difluormethoxy)-pyrimidin-2-yl]-harnstoff,
Smp. 186–188 °C.

Beispiel 3:
N-(2-Methoxycarbonylphenyl-sulfonyl)-N'-[4,6-bis-(difluormethoxy)-pyrimidin-2-yl]-N'-methyl-harnstoff

a) 67,5 g (1,0 Mol) Methylammoniumchlorid werden zusamen mit 42,0 g (0,5 Mol) Cyanguanidin auf 175 °C erhitzt. Es entsteht eine klare farblose Schmelze. Nach Einsetzen der Reaktion erwärmt sich das Reaktionsgemisch durch die freiwerdende Reaktionswärme auf 206 °C. Danach wird für 3,5 Stunden bei 175 °C gerührt, auf 80 °C abgekühlt, mit 320 ml Methanol verdünnt und innerhalb von 5 Minuten mit 172,3 g 30%iger methanolischer Natriummethylatlösung (0,97 Mol) versetzt. Die entstehende Suspension wird für 30 Minuten am Rückfluss gekocht und anschliessend innerhalb von 5 Minuten mit 173,0 g (1,08 Mol) Malonsäurediäthylester versetzt. Diese Suspension wird für weitere 6 Stunden zum Rückfluss erhitzt und dann mit 1,25 l Wasser verdünnt. Durch Ansäuern der klaren orangefarbigen Lösung mit Eisessig bis auf einen pH-Wert von 4,5 wird das Produkt ausgefällt. Der Niederschlag wird abgetrennt, mit Wasser gewaschen und bei 60 °C über Phosphorpentoxid im Vakuum getrocknet. Man erhält so 35,3 g (25,0%) 2-Methylamino-4,6-dihydroxypyrimidin, Smp. > 290 °C.

b) Ein Gemisch aus 35,3 g (0,25 Mol) 2-Methylamino-4,6-dihydroxypyrimidin, 340 g 30%iger wässriger Natriumhydroxidlösung (2,5 Mol) und 600 ml Dioxan wird auf 75 °C erhitzt. In diese Emulsion leitet man für 1,5 Stunden gasförmiges Difluorchlormethan ein. Die organische Phase wird abgetrennt und eingedampft. Der Rückstand wird mit Eiswasser gewaschen und getrocknet. Man erhält so das gewünschte 2-Methylamino-4,6-bis-(difluormethoxy)-pyrimidin, Smp. 54–55 °C.

c) Analog zu Beispiel 1b erhält man aus 2,6 g (0,011 Mol) 2-Methoxycarbonylphenyl-sulfonylisocyanat und 2,4 g (0,01 Mol) 2-Methylamino-4,6-bis-(difluormethoxy)-pyrimidin in 30 ml Methylenchlorid 4,6 g (95,8%)
N-(2-Methoxycarbonylphenyl-sulfonyl)-N'-[4,6-bis-(difluormethoxy)-pyrimidin-2-yl]-N'-methyl-harnstoff,
der nach dem Umkristallisieren aus Aceton/Äther-Gemisch bei 114–115 °C schmilzt.

In analoger Weise erhält man die in den anschliessenden Tabellen aufgeführten Verbindungen.

Tabelle 1:

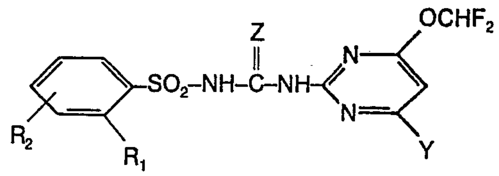

| Nr. | $R_1$ | $R_2$ | Y | Z | Smp. |
|---|---|---|---|---|---|
| 1.1 | $-COOCH_3$ | H | $CH_3$ | O | 163–164 °C |
| 1.2 | $-COOCH_3$ | H | $-OCHF_2$ | O | 186–188 °C |
| 1.3 | $-COOCH_3$ | H | $C_2H_5$ | O | 170–171 °C |
| 1.4 | $-COOCH_3$ | H | $OCH_3$ | O | 177–178 °C |
| 1.5 | Cl | H | $CH_3$ | O | 169–170 °C |
| 1.6 | Cl | H | $C_2H_5$ | O | |
| 1.7 | Cl | H | $-OCHF_2$ | O | 181–182 °C |
| 1.8 | $CH_3$ | H | $CH_3$ | O | 168–171 °C |
| 1.9 | $CH_3$ | H | $-OCHF_2$ | O | |
| 1.10 | $OCH_3$ | H | $CH_3$ | O | 164–165 °C |
| 1.11 | $OCH_3$ | H | $-OCHF_2$ | O | 183–184 °C |
| 1.12 | $OCH_3$ | H | $OCH_3$ | O | 173–174 °C |
| 1.13 | $CF_3$ | H | $OCHF_2$ | O | |
| 1.14 | $CF_3$ | H | $CH_3$ | O | 203–204 °C (Zers.) |
| 1.15 | Br | H | $-OCHF_2$ | O | |
| 1.16 | Br | H | $CH_3$ | O | 170–171 °C |
| 1.17 | Br | H | $C_2H_5$ | O | |
| 1.18 | $NO_2$ | H | $CH_3$ | O | 194–195 °C (Zers.) |
| 1.19 | $NO_2$ | H | $-OCHF_2$ | O | 173–174 °C |
| 1.20 | $NO_2$ | H | $OCH_3$ | O | 182–184 °C |
| 1.21 | $OCH_3$ | $5-OCH_3$ | $CH_3$ | O | 177–178 °C |
| 1.22 | $OCH_3$ | $5-OCH_3$ | $-OCHF_2$ | O | |
| 1.23 | $-CO-SCH_3$ | H | $-OCHF_2$ | O | |
| 1.24 | $-CO-SCH_3$ | H | $CH_3$ | O | |
| 1.25 | $-CO-N(CH_3)_2$ | H | $CH_3$ | O | |
| 1.26 | $-CO-N(CH_3)_2$ | H | $-OCHF_2$ | O | |
| 1.27 | $-CO-N(CH_3)_2$ | H | $-N(CH_3)_2$ | O | |
| 1.28 | $-CO-N(CH_3)OCH_3$ | H | $CH_3$ | O | |
| 1.29 | $-CO-N(CH_3)OCH_3$ | H | $-OCHF_2$ | O | |
| 1.30 | $-CHO$ | H | $CH_3$ | O | |
| 1.31 | $-CHO$ | H | $-OCHF_2$ | O | |
| 1.32 | $-CHO$ | H | $C_2H_5$ | O | |
| 1.33 | $-SO_2-(CH_2)_2-CH_3$ | H | $CH_3$ | O | 169–170 °C |
| 1.34 | $-SO_2-(CH_2)_2-CH_3$ | H | $-OCHF_2$ | O | |
| 1.35 | $-SO_2-CH(CH_3)_2$ | H | $CH_3$ | O | |
| 1.36 | $-SO_2-CH(CH_3)_2$ | H | $-OCHF_2$ | O | |
| 1.37 | $-SO_2-CH(CH_3)_2$ | H | $OCH_3$ | O | |
| 1.38 | $-SO_2-CH(CH_3)_2$ | H | $-N(CH_3)_2$ | O | |
| 1.39 | $-SO_2-N(CH_3)_2$ | $5-CF_3$ | $-OCHF_2$ | O | |
| 1.40 | $-SO_2-N(CH_3)_2$ | $5-CF_3$ | $CH_3$ | O | |
| 1.41 | $-COOCH_3$ | H | $-N(CH_3)C_2H_5$ | O | |
| 1.42 | Cl | H | $-N(CH_3)_2$ | O | 185–187 °C |
| 1.43 | $NO_2$ | H | $-N(CH_3)_2$ | O | 198–199 °C |
| 1.44 | $OCH_3$ | H | $-N(CH_3)_2$ | O | |
| 1.45 | $CF_3$ | H | $-N(CH_3)_2$ | O | |
| 1.46 | $OCH_3$ | $5-OCH_3$ | $-N(CH_3)_2$ | O | |
| 1.47 | $-CO-CH_3$ | H | $-N(CH_3)_2$ | O | |
| 1.48 | $NH_2$ | H | $-N(CH_3)_2$ | O | |
| 1.49 | $-COOCH_3$ | 5–F | $CH_3$ | O | |
| 1.50 | $-COOCH_3$ | 5–F | $-OCHF_2$ | O | |
| 1.51 | $-COOCH_3$ | 5–F | $-N(CH_3)_2$ | O | |
| 1.52 | $-COOCH_3$ | 6–F | $CH_3$ | O | |

Tabelle 1: (Fortsetzung)

| Nr. | $R_1$ | $R_2$ | Y | Z | Smp. |
|---|---|---|---|---|---|
| 1.53 | $-COOCH_3$ | 6–F | $-OCHF_2$ | O | |
| 1.54 | $-COOCH_3$ | 6–F | $-N(CH_3)_2$ | O | |
| 1.55 | $OCH_3$ | 6–Cl | $CH_3$ | O | 163–164 °C |
| 1.56 | $OCH_3$ | 6–Cl | $-OCHF_2$ | O | |
| 1.57 | $OCH_3$ | 6–Cl | $-N(CH_3)_2$ | O | |
| 1.58 | $-COOCH_3$ | 5–Cl | $CH_3$ | O | |
| 1.59 | $-COOCH_3$ | 5–Cl | $-OCHF_2$ | O | |
| 1.60 | $-COOCH_3$ | 5–Cl | $C_2H_5$ | O | |
| 1.61 | $NO_2$ | H | $CH_3$ | S | |
| 1.62 | $NO_2$ | H | $-OCHF_2$ | S | |
| 1.63 | $-SO_2-N(CH_3)_2$ | H | $CH_3$ | S | |
| 1.64 | $-SO_2-N(CH_3)_2$ | H | $-OCHF_2$ | S | |
| 1.65 | $-CO-CH_3$ | H | $CH_3$ | S | |
| 1.66 | $-CO-CH_3$ | H | $-OCHF_2$ | S | |
| 1.67 | $-COOCH(CH_3)_2$ | H | $CH_3$ | O | 169–170 °C |
| 1.68 | $-COOCH(CH_3)_2$ | H | $-OCHF_2$ | O | |
| 1.69 | $-COOCH(CH_3)_2$ | H | $OCH_3$ | O | 178–180 °C |
| 1.70 | $-COOCH_2-CH=CH_2$ | H | $CH_3$ | O | |
| 1.71 | $-COOCH_2-CH=CH_2$ | H | $-OCHF_2$ | O | |
| 1.72 | $-COOCH_2-CH=CH_2$ | H | $C_2H_5$ | O | |
| 1.73 | $-COOCH_2-CF_3$ | H | $CH_3$ | O | |
| 1.74 | $-COOCH_2-CF_3$ | H | $-OCHF_2$ | O | |
| 1.75 | $-COOCH_2-OCH_3$ | H | $CH_3$ | O | |
| 1.76 | $-COOCH_2-OCH_3$ | H | $-OCHF_2$ | O | |
| 1.77 | $-COOCH_3$ | H | $-OCH_2-CH_3$ | O | 153–159 °C |
| 1.78 | $-COOCH_3$ | H | $-N(CH_3)_2$ | O | 229–230 °C |
| 1.79 | $NH_2$ | H | $CH_3$ | O | 222–225 °C (Zers.) |
| 1.80 | $-NH-CO-CH_3$ | H | $CH_3$ | O | 171–172 °C |
| 1.81 | $NH_2$ | H | $-OCHF_2$ | O | |
| 1.82 | $-NH-CO-CH_3$ | H | $-OCHF_2$ | O | |
| 1.83 | $SCH_3$ | H | $CH_3$ | O | 168–169 °C |
| 1.84 | $SCH_3$ | H | $-OCHF_2$ | O | 187–188 °C |
| 1.85 | $-SO_2-CH_3$ | H | $CH_3$ | O | 208 °C (Zers.) |
| 1.86 | $-SO_2-CH_3$ | H | $-OCHF_2$ | O | |
| 1.87 | CN | H | $CH_3$ | O | |
| 1.88 | CN | H | $-OCHF_2$ | O | |
| 1.89 | CN | H | $OCH_3$ | O | |
| 1.90 | $-CO-CH_3$ | H | $CH_3$ | O | |
| 1.91 | $-CO-CH_3$ | H | $-OCHF_2$ | O | |
| 1.92 | $-CO-CH_3$ | H | $OCH_3$ | O | |
| 1.93 | $-SO_2-N(CH_3)_2$ | H | $CH_3$ | O | 196–198 °C |
| 1.94 | $-SO_2-N(CH_3)_2$ | H | $-OCHF_2$ | O | 170–172 °C |
| 1.95 | F | H | $CH_3$ | O | 158–159 °C |
| 1.96 | F | H | $-OCHF_2$ | O | 200–201 °C |
| 1.97 | Cl | H | $-OCHF_2$ | S | 157–159 °C |
| 1.98 | Cl | H | $CH_3$ | S | 167 °C (Zers.) |
| 1.99 | $-COOCH_3$ | H | $CHF_2$ | O | |
| 1.100 | $-COOCH_3$ | H | $CH_2F$ | O | 164–165 °C |
| 1.101 | $-COOCH_3$ | H | $CF_3$ | O | 165–166 °C |
| 1.102 | $-COOCH_3$ | H | Cl | O | 171–172 °C |
| 1.103 | $-COOCH_3$ | H | $-OCH_2-CH_2F$ | O | |
| 1.104 | $-COOCH_3$ | H | $-OCH_2-CF_3$ | O | |
| 1.105 | $-COOCH_3$ | H | $-NH-CH_3$ | O | |
| 1.106 | $-COOCH_3$ | H | Br | O | |
| 1.107 | $-COOCH_3$ | H | $CH_2Cl$ | O | |
| 1.108 | $-COOCH_3$ | H | F | O | |
| 1.109 | $NO_2$ | H | Cl | O | 190–191 °C |
| 1.110 | $NO_2$ | H | $CF_3$ | O | 175–176 °C |

Tabelle 1: (Fortsetzung)

| Nr. | $R_1$ | $R_2$ | Y | Z | Smp. |
|---|---|---|---|---|---|
| 1.111 | $NO_2$ | H | $CH_2F$ | O | |
| 1.112 | $NO_2$ | H | $CH_2Cl$ | O | |
| 1.113 | $OCH_3$ | H | Cl | O | 142–144 °C |
| 1.114 | $OCH_3$ | H | $CF_3$ | O | 153–154 °C |
| 1.115 | $OCH_3$ | H | $CH_2F$ | O | |
| 1.116 | $NH_2$ | H | $OCH_3$ | O | |
| 1.117 | $NH_2$ | H | Cl | O | |
| 1.118 | Br | H | $CH_2F$ | O | |
| 1.119 | Br | H | $OCH_3$ | O | |
| 1.120 | Br | H | $-N(CH_3)_2$ | O | |
| 1.121 | Br | H | Cl | O | |
| 1.122 | $-COOCH_3$ | 3–Cl | $CH_3$ | O | |
| 1.123 | $-COOCH_3$ | 3–Cl | $OCH_3$ | O | |
| 1.124 | $-COOCH_3$ | 3–Cl | $OCHF_2$ | O | |
| 1.125 | $-COOCH_3$ | 3–Cl | $-N(CH_3)_2$ | O | |
| 1.126 | $-COOCH_3$ | 3–F | $CH_3$ | O | |
| 1.127 | $-COOCH_3$ | 3–F | $OCH_3$ | O | |
| 1.128 | $-COOCH_3$ | 3–F | $OCHF_2$ | O | |
| 1.129 | $-COOCH_3$ | 3–F | $-N(CH_3)_2$ | O | |
| 1.130 | $CH_3$ | 6–Cl | $CH_3$ | O | 173–176 °C |
| 1.131 | $CH_3$ | 6–Cl | $OCH_3$ | O | |
| 1.132 | $CH_3$ | 6–Cl | $OCHF_2$ | O | |
| 1.133 | $CH_3$ | 6–Cl | $-N(CH_3)_2$ | O | |
| 1.134 | $NO_2$ | 3–Cl | $CH_3$ | O | |
| 1.135 | $CH_3$ | 6–$CH_3$ | $CH_3$ | O | |
| 1.136 | $CH_3$ | 6–$CH_3$ | $OCH_3$ | O | |
| 1.137 | $-COOCH_3$ | 6–$CH_3$ | $CH_3$ | O | |
| 1.138 | $-COOCH_3$ | 3–$CH_3$ | $CH_3$ | O | 173–175 °C (Zers.) |
| 1.139 | $OCH_3$ | H | $C_2H_5$ | O | |
| 1.140 | $OCH_3$ | 5–F | $CH_3$ | O | 173–175 °C (Zers.) |
| 1.141 | $OCH_3$ | 5–F | $OCHF_2$ | O | |
| 1.142 | $OCH_3$ | 5–F | $OCH_3$ | O | |
| 1.143 | $OCH_3$ | 5–F | $-N(CH_3)_2$ | O | |
| 1.144 | $OCH_3$ | 5–Cl | $CH_3$ | O | 176–177 °C (Zers.) |
| 1.145 | $OCH_3$ | 5–Cl | $OCH_3$ | O | |
| 1.146 | J | H | $CH_3$ | O | 164–166 °C |
| 1.147 | $OCH_3$ | H | $C_2H_5$ | O | 170–171 °C |
| 1.148 | H | H | $CH_3$ | O | 176–177 °C |
| 1.149 | Cl | H | $OCH_3$ | O | 172–173 °C |
| 1.150 | $CH_3$ | 5–$NO_2$ | $CH_3$ | O | 189–190 °C |
| 1.151 | $CH_3$ | 5–$NH_2$ | $CH_3$ | O | 133–135 °C |
| 1.152 | $CH_3$ | H | Cl | O | |
| 1.153 | $CH_3$ | H | $OCH_3$ | O | |
| 1.154 | $CH_3$ | H | $-N(CH_3)_2$ | O | |
| 1.155 | $-NH-CO-CH_3$ | H | $OCH_3$ | O | |
| 1.156 | $OCH_3$ | 5–$OCH_3$ | $OCH_3$ | O | |
| 1.157 | $-CO-SCH_3$ | H | $OCH_3$ | O | |
| 1.158 | $-CO-SCH_3$ | H | $-N(CH_3)$ | O | |
| 1.159 | $-CO-SCH_3$ | H | Cl | O | |
| 1.160 | $-CO-N(CH_3)_2$ | H | $OCH_3$ | O | |
| 1.161 | $-CO-N(CH_3)_2$ | H | Cl | O | |
| 1.162 | $-SO_2-C_3H_7-n$ | H | $OCH_3$ | O | |
| 1.163 | $-SO_2-C_3H_7-n$ | H | $-N(CH_3)_2$ | O | |
| 1.164 | $-SO_2-C_3H_7-n$ | H | Cl | O | |
| 1.165 | $-SO_2-C_3H_7-n$ | H | Cl | O | |
| 1.166 | $-SO_2-C_2H_5$ | H | $CH_3$ | O | |

Tabelle 1: (Fortsetzung)

| Nr. | $R_1$ | $R_2$ | Y | Z | Smp. |
|---|---|---|---|---|---|
| 1.167 | $-SO_2-C_2H_5$ | H | $-OCHF_2$ | O | |
| 1.168 | $-SO_2C_2H_5$ | H | $OCH_3$ | O | |
| 1.169 | $-SO_2C_2H_5$ | H | $-N(CH_3)_2$ | O | |
| 1.170 | $-SO_2C_2H_5$ | H | Cl | O | |
| 1.171 | $-SO_2-CH_3$ | H | $OCH_3$ | O | |
| 1.172 | $-SO_2-CH_3$ | H | $-N(CH_3)_2$ | O | |
| 1.173 | $-SO_2CH_3$ | H | Cl | O | |
| 1.174 | Cl | H | Cl | O | |
| 1.175 | $OCH_3$ | $5-OCH_3$ | Cl | O | |
| 1.176 | $SCH_3$ | H | $OCH_3$ | O | 181–182 °C |
| 1.177 | $SCH_3$ | H | Cl | O | |
| 1.178 | $SCH_3$ | H | $-N(CH_3)_2$ | O | |
| 1.179 | $-SO_2-N(CH_3)_2$ | H | $OCH_3$ | O | |
| 1.180 | $-SO_2-N(CH_3)_2$ | H | Cl | O | |
| 1.181 | $-SO_2N(CH_3)_2$ | H | $-N(CH_3)_2$ | O | |
| 1.182 | $-COOCH_3$ | 6–F | $OCH_3$ | O | |
| 1.183 | $-CO-CH_3$ | H | Cl | O | |
| 1.184 | $-COOCH_3$ | 6–F | Cl | O | |
| 1.185 | $OCH_3$ | 6–Cl | $OCH_3$ | O | |
| 1.186 | $OCH_3$ | 6–Cl | Cl | O | |
| 1.187 | $-COOCH_3$ | 5–Cl | $OCH_3$ | O | |
| 1.188 | $-COOCH_3$ | 5–Cl | Cl | O | |
| 1.189 | CN | H | $-N(CH_3)_2$ | O | |
| 1.190 | F | H | $OCH_3$ | O | 198–199 °C |
| 1.191 | F | H | $-N(CH_3)_2$ | O | |
| 1.192 | F | H | Cl | O | |
| 1.193 | J | H | $OCHF_2$ | O | 174–176 °C (Zers.) |
| 1.194 | J | H | $OCH_3$ | O | 134–136 °C (Zers.) |
| 1.195 | J | H | $-N(CH_3)_2$ | O | 234 °C (Zers.) |
| 1.196 | H | H | $OCHF_2$ | O | |
| 1.197 | H | H | $OCH_3$ | O | |
| 1.198 | H | H | $-N(CH_3)_2$ | O | |
| 1.199 | H | H | $-N(CH_3)_2$ | O | |
| 1.200 | $NO_2$ | 5–F | $CH_3$ | O | |
| 1.201 | $NO_2$ | 5–Cl | $CH_3$ | O | |
| 1.202 | $OCH_3$ | $6-SCH_3$ | $CH_3$ | O | 140–143 °C |
| 1.203 | $OCH_3$ | $6-SCH_3$ | $OCH_3$ | O | |
| 1.204 | $OCH_3$ | $6-SCH_3$ | $OCHF_2$ | O | |
| 1.205 | $-S-C_3H_7-i$ | H | $CH_3$ | O | |
| 1.206 | $-S-C_3H_7-i$ | H | $OCH_3$ | O | |
| 1.207 | $-S-C_3H_7-i$ | H | $OCHF_2$ | O | |
| 1.208 | $-O-C_2H_5$ | H | $CH_3$ | O | 188–189 °C |
| 1.209 | $-O-C_2H_5$ | H | $OCH_3$ | O | 172–173 °C |
| 1.210 | $-O-C_2H_5$ | H | $OCHF_2$ | O | 165–166 °C |
| 1.211 | $-O-C_2H_5$ | H | $-N(CH_3)_2$ | O | 195–196 °C |
| 1.212 | $-O-C_2H_5$ | H | Cl | O | |
| 1.213 | $-O-C_3H_7-i$ | H | $CH_3$ | O | 166–167 °C |
| 1.214 | $-O-C_3H_7-i$ | H | $OCH_3$ | O | 168–170 °C |
| 1.215 | $-O-C_3H_7-i$ | H | $OCHF_2$ | O | 147–148 °C |
| 1.216 | $-O-C_3H_7-i$ | H | $-N(CH_3)_2$ | O | |
| 1.217 | $-O-C_3H_7-i$ | H | Cl | O | |
| 1.218 | $-O-C_3H_7-n$ | H | $CH_3$ | O | |
| 1.219 | $C_2H_5$ | H | $CH_3$ | O | |
| 1.220 | $C_2H_5$ | H | $OCH_3$ | O | |
| 1.221 | $C_2H_5$ | H | $OCHF_2$ | O | |
| 1.222 | $C_2H_5$ | H | $-N(CH_3)_2$ | O | |
| 1.223 | $C_2H_5$ | H | Cl | O | |

Tabelle 1: (Fortsetzung)

| Nr. | $R_1$ | $R_2$ | Y | Z | Smp. |
|---|---|---|---|---|---|
| 1.224 | $C_2H_5$ | H | $CH_2F$ | O | |
| 1.225 | $C_2H_5$ | H | $CF_3$ | O | |
| 1.226 | $-COOCH_3$ | H | $-O-C_3H_7-i$ | O | |
| 1.227 | $NO_2$ | H | $-O-C_3H_7-i$ | O | |
| 1.228 | $NO_2$ | H | $-O-C_2H_5$ | O | |
| 1.229 | $NO_2$ | H | $-O-CH_2-CF_3$ | O | |
| 1.230 | $-COOCH_3$ | H | $-O-CH_2-CF_3$ | O | |
| 1.231 | $-COOCH_3$ | H | $SCH_3$ | O | |
| 1.232 | $NO_2$ | H | $SCH_3$ | O | |
| 1.233 | $CF_3$ | H | $SCH_3$ | O | |
| 1.234 | $-SO_2-N(CH_3)_2$ | H | $SCH_3$ | O | |
| 1.235 | $OCH_3$ | H | $SCH_3$ | O | |
| 1.236 | $-COOCH_3$ | H | $-CH_2-OCH_3$ | O | |
| 1.237 | $NO_2$ | H | $-CH_2-OCH_3$ | O | |
| 1.238 | $-COOCH_3$ | H | $-CH_2-OC_2H_5$ | O | |
| 1.239 | $NO_2$ | H | $-CH_2-OC_2H_5$ | O | |
| 1.240 | $-CH_2-CH_2-CF_3$ | H | $CH_3$ | O | 164–165 °C |
| 1.241 | $-CH_2-CH_2-CF_3$ | H | $OCH_3$ | O | |
| 1.242 | $-CH_2-CH_2-CF_3$ | H | $OCHF_2$ | O | |
| 1.243 | $-CH_2-CH_2-CF_3$ | H | Cl | O | |
| 1.244 | $-CH_2-CH_2CF_3$ | H | $-N(CH_3)_2$ | O | |
| 1.245 | $CF_3$ | H | $OCH_3$ | O | |
| 1.246 | $CF_3$ | H | Cl | O | |
| 1.247 | $CF_3$ | H | $-OC_2H_5$ | O | |
| 1.248 | $NH_2$ | H | $OCH_3$ | O | |
| 1.249 | $NH_2$ | H | Cl | O | |
| 1.250 | $-N(CH_3)_2$ | H | $CH_3$ | O | |
| 1.251 | $-N(CH_3)_2$ | H | $OCH_3$ | O | |
| 1.252 | $-N(CH_3)_2$ | H | $OCHF_2$ | O | |
| 1.253 | $-N(CH_3)_2$ | H | $-N(CH_3)_2$ | O | |
| 1.254 | $-N(CH_3)_2$ | H | Cl | O | |
| 1.255 | $NO_2$ | H | $OCH_3$ | S | |
| 1.256 | $-CO-NH-CH_3$ | H | $CH_3$ | O | |
| 1.257 | $-CO-NH-CH_3$ | H | $OCH_3$ | O | |
| 1.258 | $-CO-NH-CH_3$ | H | $OCHF_2$ | O | |
| 1.259 | $-CO-NH_2$ | H | $CH_3$ | O | |
| 1.260 | $-CO-NH_2$ | H | $OCH_3$ | O | |
| 1.261 | $-CO-NH_2$ | H | $OCHF_2$ | O | |
| 1.262 | CHO | H | $OCH_3$ | O | |
| 1.263 | CHO | H | $-N(CH_3)_2$ | O | |
| 1.264 | $-CO-CF_3$ | H | $CH_3$ | O | |
| 1.265 | $-CO-CF_3$ | H | $OCH_3$ | O | |
| 1.266 | $-CO-CF_3$ | H | $OCHF_2$ | O | |
| 1.267 | $-SO-CH_3$ | H | $CH_3$ | O | |
| 1.268 | $-SO-CH_3$ | H | $OCH_3$ | O | |
| 1.269 | $-SO-CH_3$ | H | $OCHF_2$ | O | |
| 1.270 | $OCH_3$ | H | $-OC_2H_5$ | O | |
| 1.271 | $-OC_3H_7-n$ | H | $OCH_3$ | O | |
| 1.272 | $-OC_3H_7-n$ | H | $OCHF_2$ | O | |
| 1.273 | $-OC_3H_7-n$ | H | $-N(CH_3)_2$ | O | |
| 1.274 | $-OC_3H_7-n$ | H | Cl | O | |
| 1.275 | $-OC_4H_9-n$ | H | $CH_3$ | O | |
| 1.276 | $-OC_4H_9-n$ | H | $OCH_3$ | O | |
| 1.277 | $-OC_4H_9-n$ | H | $OCHF_2$ | O | |
| 1.278 | $-COO-C_4H_9-s$ | H | $CH_3$ | O | 168–169 °C |
| 1.279 | $-COO-C_4H_9-s$ | H | $OCH_3$ | O | 164–166 °C |
| 1.280 | $-COO-C_4H_9-s$ | H | $OCHF_2$ | O | 167–169 °C |
| 1.281 | $-COO-CH(CH_3)CH_2-OCH_3$ | H | $CH_3$ | O | |
| 1.282 | $-COO-CH(CH_3)CH_2-OCH_3$ | H | $OCH_3$ | O | |

Tabelle 1: (Fortsetzung)

| Nr. | $R_1$ | $R_2$ | Y | Z | Smp. |
|---|---|---|---|---|---|
| 1.283 | $-COO-CH(CH_3)CH_2-OCH_3$ | H | $OCHF_2$ | O | |
| 1.284 | $-COO-C_4H_9-i$ | H | $CH_3$ | O | 125–130 °C |
| 1.285 | $-COO-C_4H_9-i$ | H | $OCH_3$ | O | 167–168 °C |
| 1.286 | $-COO-C_4H_9-i$ | H | $OCHF_2$ | O | |
| 1.287 | $-COO-C_5H_{11}-n$ | H | $CH_3$ | O | |
| 1.288 | $-COO-C_5H_{11}-n$ | H | $OCH_3$ | O | |
| 1.289 | $-COO-C_5H_{11}-n$ | H | $OCHF_2$ | O | |
| 1.290 | $-COO-CH_2-CH=CH_2$ | H | $OCH_3$ | O | |
| 1.291 | $-COO-CH_2-CF_3$ | H | $OCH_3$ | O | |
| 1.292 | $-COO-CH_2-C_6H_5$ | H | $CH_3$ | O | |
| 1.293 | $-COO-CH_2-C_6H_5$ | H | $OCH_3$ | O | |
| 1.294 | $-COO-CH_2-C_6H_5$ | H | $OCHF_2$ | O | |
| 1.295 | $-COO-CH_2-C\equiv CH$ | H | $CH_3$ | O | |
| 1.296 | $-COO-CH_2-C\equiv CH$ | H | $OCH_3$ | O | |
| 1.297 | $-COO-CH_2-C\equiv CH$ | H | $OCHF_2$ | O | |
| 1.298 | $-COO-CH_3$ | H | $CH_3$ | S | |
| 1.299 | $-COO-CH_3$ | H | $OCH_3$ | S | |
| 1.300 | $-COO-CH_3$ | H | $OCHF_2$ | S | |
| 1.301 | $-COO-CH_3$ | H | $-N(CH_3)_2$ | S | |
| 1.302 | $-COO-CH_3$ | H | $-CH_2Cl$ | O | |
| 1.303 | $-COO-CH_3$ | 6–Cl | $CH_3$ | O | |
| 1.304 | $-COO-CH_3$ | 6–Cl | $OCH_3$ | O | |
| 1.305 | $-COO-CH_3$ | 6–Cl | $OCHF_2$ | O | |
| 1.306 | $-COO-CH_3$ | 6–Cl | $-N(CH_3)_2$ | O | |
| 1.307 | $-COO-CH_3$ | 6–Cl | Cl | O | |
| 1.308 | $-COO-CH_3$ | 6–Cl | $CH_2F$ | O | |
| 1.309 | $-COO-CH_3$ | 6–Cl | $-OC_2H_5$ | O | |
| 1.310 | $-COO-C_2H_5$ | H | $CH_3$ | O | 150–152 °C |
| 1.311 | $-COO-C_2H_5$ | H | $OCH_3$ | O | |
| 1.312 | $-COO-C_2H_5$ | H | $OCHF_2$ | O | |
| 1.313 | $-COO-C_2H_5$ | H | $-N(CH_3)_2$ | O | |
| 1.314 | $-COO-C_2H_5$ | H | Cl | O | |
| 1.315 | $-COO-C_2H_5$ | H | $CH_2F$ | O | |
| 1.316 | $-COO-C_3H_7-i$ | H | $-N(CH_3)_2$ | O | |
| 1.317 | $-COO-CH_2CH_2-OCH_3$ | H | $CH_3$ | O | 150–152 °C |
| 1.318 | $-COO-CH_2CH_2-OCH_3$ | H | $OCH_3$ | O | |
| 1.319 | $-COO-CH_2CH_2-OCH_3$ | H | $OCHF_2$ | O | |
| 1.320 | $-COO-CH_2CH_2-OCH_3$ | H | $-N(CH_3)_2$ | O | |
| 1.321 | $-COO-CH_2CH_2-OCH_3$ | H | Cl | O | |
| 1.322 | $-COO-CH_2-CH_2-Cl$ | H | $CH_3$ | O | |
| 1.323 | $-COO-CH_2-CH_2-Cl$ | H | $OCH_3$ | O | |
| 1.324 | $-COO-CH_2-CH_2-Cl$ | H | $OCHF_2$ | O | |
| 1.325 | $-COO-CH_2-CH_2-Cl$ | H | $-N(CH_3)_2$ | O | |
| 1.326 | $-COO-CH_2-CH_2-Cl$ | H | Cl | O | |
| 1.327 | $C_3H_7-n$ | H | $CH_3$ | O | 147–148 °C (Zers.) |
| 1.328 | $C_3H_7-n$ | H | $OCH_3$ | O | |
| 1.329 | $C_3H_7-n$ | H | $OCHF_2$ | O | |
| 1.330 | $C_3H_7-n$ | H | $-N(CH_3)_2$ | O | |
| 1.331 | $C_3H_7-n$ | H | Cl | O | |
| 1.332 | $C_3H_7-n$ | H | $CH_2F$ | O | |
| 1.333 | H | $3-NO_2$ | $CH_3$ | O | 190–193 °C |
| 1.334 | H | $3-NO_2$ | $OCH_3$ | O | |
| 1.335 | H | $3-NO_2$ | $OCHF_2$ | O | |
| 1.336 | $CH_2Cl$ | H | $CH_3$ | O | 153–156 °C |
| 1.337 | $CH_2Cl$ | H | $OCH_3$ | O | |
| 1.338 | $CH_2Cl$ | H | $OCHF_2$ | O | |
| 1.339 | $CH_2Cl$ | H | $-N(CH_3)_2$ | O | |
| 1.340 | $CH_2Cl$ | H | Cl | O | |

Tabelle 1: (Fortsetzung)

| Nr. | $R_1$ | $R_2$ | Y | Z | Smp. |
|---|---|---|---|---|---|
| 1.341 | $CH_2Cl$ | H | $CH_2F$ | O | |
| 1.342 | $-SO_2-O-CH_2CF_3$ | H | $CH_3$ | O | 176–179 °C |
| 1.343 | $-SO_2-O-CH_2-CF_3$ | H | $OCH_3$ | O | |
| 1.344 | $-SO_2-O-CH_2-CF_3$ | H | $OCHF_2$ | O | |
| 1.345 | $-SO_2-O-CH_2-CF_3$ | H | $-N(CH_3)_2$ | O | |
| 1.346 | $-SO_2-O-CH_2-CF_3$ | H | Cl | O | |
| 1.347 | $-SO_2-O-CH_2-CF_3$ | H | $CH_2F$ | O | |
| 1.348 | H | $3-COOCH_3$ | $CH_3$ | O | |
| 1.349 | H | $3-COOCH_3$ | $OCH_3$ | O | |
| 1.350 | H | $3-COOCH_3$ | $OCHF_2$ | O | |
| 1.351 | H | $3-COOCH_3$ | $-N(CH_3)_2$ | O | |
| 1.352 | $OCH_3$ | $4-NO_2$ | $CH_3$ | O | 164–167 °C |
| 1.353 | $OCH_3$ | $4-NO_2$ | $OCH_3$ | O | |
| 1.354 | $OCH_3$ | $4-NO_2$ | $OCHF_2$ | O | |
| 1.355 | $OCH_3$ | $4-NO_2$ | $-N(CH_3)_2$ | O | |
| 1.356 | $OCH_3$ | $4-NO_2$ | Cl | O | |
| 1.357 | $OCH_3$ | $4-NH_2$ | $CH_3$ | O | |
| 1.357 | $OCH_3$ | $4-NH_2$ | $OCH_3$ | O | |
| 1.358 | $OCH_3$ | $4-NH_2$ | $OCH_3$ | O | |
| 1.359 | $OCH_3$ | $4-NH_2$ | $OCHF_2$ | O | |
| 1.360 | $OCH_3$ | $4-NH_2$ | $-N(CH_3)_2$ | O | |
| 1.361 | $OCH_3$ | $4-NH_2$ | Cl | O | |
| 1.362 | $NH_2$ | $6-OCH_3$ | $CH_3$ | O | |
| 1.363 | $NH_2$ | $6-OCH_3$ | $OCH_3$ | O | |
| 1.364 | $NH_2$ | $6-OCH_3$ | $OCHF_2$ | O | |
| 1.365 | $NH_2$ | $6-OCH_3$ | $-N(CH_3)_2$ | O | |
| 1.366 | $NH_2$ | $6-OCH_3$ | Cl | O | |
| 1.367 | $OCH_3$ | $6-OCH_3$ | $CH_3$ | O | |
| 1.368 | $OCH_3$ | $6-OCH_3$ | $OCH_3$ | O | |
| 1.369 | $OCH_3$ | $6-OCH_3$ | $OCHF_2$ | O | |
| 1.370 | $OCH_3$ | $6-OCH_3$ | $-N(CH_3)_2$ | O | |
| 1.371 | $OCH_3$ | $6-OCH_3$ | Cl | O | |
| 1.372 | $OCH_3$ | $3-OCH_3$ | $CH_3$ | O | |
| 1.373 | $OCH_3$ | $3-OCH_3$ | $OCH_3$ | O | |
| 1.374 | $OCH_3$ | $3-OCH_3$ | $OCHF_2$ | O | |
| 1.375 | Cl | 6–Cl | $CH_3$ | O | 170–172 °C |
| 1.376 | Cl | 6–Cl | $OCH_3$ | O | |
| 1.377 | Cl | 6–Cl | $OCHF_2$ | O | |
| 1.378 | Cl | 6–Cl | $-N(CH_3)_2$ | O | |
| 1.379 | Cl | 6–Cl | Cl | O | |
| 1.380 | Cl | 6–Cl | $CH_2F$ | O | |
| 1.381 | $NO_2$ | 6–Cl | $CH_3$ | O | 180 °C (Zers.) |
| 1.382 | $NO_2$ | 6–Cl | $OCH_3$ | O | 157 °C (Zers.) |
| 1.383 | $NO_2$ | 6–Cl | $OCHF_2$ | O | 175–177 °C (Zers.) |
| 1.384 | $NO_2$ | 6–Cl | $-N(CH_3)_2$ | O | 185–188 °C (Zers.) |
| 1.385 | $NO_2$ | 6–Cl | Cl | O | |
| 1.386 | $NO_2$ | 6–Cl | $CH_2F$ | O | |
| 1.387 | Cl | 5–Cl | $CH_3$ | O | 207–209 °C |
| 1.388 | Cl | 5–Cl | $OCH_3$ | O | |
| 1.389 | Cl | 5–Cl | $OCHF_2$ | O | |
| 1.390 | $OCH_3$ | $5-CH_3$ | $CH_3$ | O | 164–167 °C |
| 1.391 | $OCH_3$ | $5-CH_3$ | $OCH_3$ | O | |
| 1.392 | $OCH_3$ | $5-CH_3$ | $OCHF_2$ | O | |
| 1.393 | $OCH_3$ | $5-CH_3$ | $-N(CH_3)_2$ | O | |
| 1.394 | $OCH_3$ | $5-CH_3$ | Cl | O | |
| 1.395 | Cl | 3–Cl | $CH_3$ | O | 182–184 °C |
| 1.396 | Cl | 3–Cl | $OCH_3$ | O | |

Tabelle 1: (Fortsetzung)

| Nr. | $R_1$ | $R_2$ | Y | Z | Smp. |
|---|---|---|---|---|---|
| 1.397 | Cl | 3–Cl | $OCHF_2$ | O | |
| 1.398 | Cl | 3–Cl | $-N(CH_3)_2$ | O | |
| 1.399 | Cl | 3–Cl | Cl | O | |
| 1.400 | Cl | 3–Cl | $CH_2F$ | O | |
| 1.401 | $CH_3$ | $5-NO_2$ | $CH_3$ | O | 189–190 °C |
| 1.402 | $CH_3$ | $5-NO_2$ | $OCH_3$ | O | |
| 1.403 | $CH_3$ | $5-NO_2$ | $OCHF_2$ | O | |
| 1.404 | $CH_3$ | $5-NO_2$ | $-N(CH_3)_2$ | O | |
| 1.405 | $CH_3$ | $5-NO_2$ | Cl | O | |
| 1.406 | $CH_3$ | $5-NH_2$ | $CH_3$ | O | 133–135 °C |
| 1.407 | $CH_3$ | $5-NH_2$ | $OCH_3$ | O | |
| 1.408 | $CH_3$ | $5-NH_2$ | $OCHF_2$ | O | |
| 1.409 | $CH_3$ | $5-NH_2$ | $-N(CH_3)_2$ | O | |
| 1.410 | $CH_3$ | $5-NH_2$ | Cl | O | |
| 1.411 | $NO_2$ | $6-OCH_3$ | $CH_3$ | O | |
| 1.412 | $NO_2$ | $6-OCH_3$ | $C_2H_5$ | O | |
| 1.413 | $NO_2$ | $6-OCH_3$ | $CH_2F$ | O | |
| 1.414 | $NO_2$ | $6-OCH_3$ | $CF_3$ | O | |
| 1.415 | $NO_2$ | $6-OCH_3$ | $OCH_3$ | O | |
| 1.416 | $NO_2$ | $6-OCH_3$ | $OC_2H_5$ | O | |
| 1.417 | $NO_2$ | $6-OCH_3$ | $OCHF_2$ | O | |
| 1.418 | $NO_2$ | $6-OCH_3$ | Cl | O | |
| 1.419 | $NO_2$ | $6-OCH_3$ | $-N(CH_3)_2$ | O | |
| 1.420 | $NO_2$ | $6-OCH_3$ | $CH_3$ | S | |
| 1.421 | $NH_2$ | 6–Cl | $CH_3$ | O | 123 °C (Zers.) |
| 1.422 | $NH_2$ | 6–Cl | $C_2H_5$ | O | |
| 1.423 | $NH_2$ | 6–Cl | $CH_2F$ | O | |
| 1.424 | $NH_2$ | 6–Cl | $CF_3$ | O | |
| 1.425 | $NH_2$ | 6–Cl | $OCH_3$ | O | |
| 1.426 | $NH_2$ | 6–Cl | $OC_2H_5$ | O | |
| 1.427 | $NH_2$ | 6–Cl | $OCHF_2$ | O | |
| 1.428 | $NH_2$ | 6–Cl | Cl | O | |
| 1.429 | $NH_2$ | 6–Cl | $-N(CH_3)_2$ | O | |
| 1.430 | F | 6–Cl | $CH_3$ | O | |
| 1.431 | F | 6–Cl | $CH_2F$ | O | |
| 1.432 | F | 6–Cl | $CF_3$ | O | |
| 1.433 | F | 6–Cl | $OCH_3$ | O | |
| 1.434 | F | 6–Cl | $OCHF_2$ | O | |
| 1.435 | F | 6–Cl | Cl | O | |
| 1.436 | F | 6–Cl | $-N(CH_3)_2$ | O | |
| 1.437 | F | 6–Cl | $CH_3$ | S | |
| 1.438 | F | 6–Cl | $OCH_3$ | S | |
| 1.439 | F | 6–Cl | $OCHF_2$ | S | |
| 1.440 | F | 6–Cl | $-N(CH_3)_2$ | S | |
| 1.441 | Br | 6–Cl | $CH_3$ | O | |
| 1.442 | Br | 6–Cl | $CH_2F$ | O | |
| 1.443 | Br | 6–Cl | $CF_3$ | O | |
| 1.444 | Br | 6–Cl | $OCH_3$ | O | |
| 1.445 | Br | 6–Cl | $OCHF_2$ | O | |
| 1.446 | Br | 6–Cl | Cl | O | |
| 1.447 | Br | 6–Cl | $-N(CH_3)_2$ | O | |
| 1.448 | J | 6–Cl | $CH_3$ | O | 129–131 °C (Zers.) |
| 1.449 | J | 6–Cl | $CH_2F$ | O | |
| 1.450 | J | 6–Cl | $CF_3$ | O | |
| 1.451 | J | 6–Cl | $OCH_3$ | O | |
| 1.452 | J | 6–Cl | $OCHF_2$ | O | 138 °C (Zers.) |
| 1.453 | J | 6–Cl | $-N(CH_3)_2$ | O | |
| 1.454 | J | 6–Cl | $CH_3$ | S | |

Tabelle 1: (Fortsetzung)

| Nr. | $R_1$ | $R_2$ | Y | Z | Smp. |
|---|---|---|---|---|---|
| 1.455 | Br | 6–Cl | $CH_3$ | S | |
| 1.456 | $-COOCH_3$ | 6–$CH_3$ | $C_2H_5$ | O | |
| 1.457 | $-COOCH_3$ | 6–$CH_3$ | $CH_2F$ | O | |
| 1.458 | $-COOCH_3$ | 6–$CH_3$ | $CF_3$ | O | |
| 1.459 | $-COOCH_3$ | 6–$CH_3$ | $OCH_3$ | O | |
| 1.460 | $-COOCH_3$ | 6–$CH_3$ | $OCHF_2$ | O | |
| 1.461 | $-COOCH_3$ | 6–$CH_3$ | Cl | O | |
| 1.462 | $-COOCH_3$ | 6–$CH_3$ | $OC_2H_5$ | O | |
| 1.463 | $-COOCH_3$ | 6–$CH_3$ | $-N(CH_3)_2$ | O | |
| 1.464 | $-COO-C_2H_5$ | 6–$CH_3$ | $CH_3$ | O | |
| 1.465 | $-COO-C_2H_5$ | 6–$CH_3$ | $OCH_3$ | O | |
| 1.466 | $-COO-C_2H_5$ | 6–$CH_3$ | $OCHF_2$ | O | |
| 1.467 | $-COO-C_2H_5$ | 6–$CH_3$ | $-N(CH_3)_2$ | O | |
| 1.468 | $-COO-C_3H_7$–i | 6–$CH_3$ | $CH_3$ | O | |
| 1.469 | $-COO-C_3H_7$–i | 6–$CH_3$ | $OCH_3$ | O | |
| 1.470 | $-COO-C_3H_7$–i | 6–$CH_3$ | $OCHF_2$ | O | |
| 1.471 | $-COO-C_3H_7$–i | 6–$CH_3$ | $-N(CH_3)_2$ | O | |
| 1.472 | $-COOCH_3$ | 6–$CH_3$ | $CH_3$ | S | |
| 1.473 | $-COOCH_3$ | 6–$CH_3$ | $OCH_3$ | S | |
| 1.474 | $-COOCH_3$ | 6–$CH_3$ | $OCHF_2$ | S | |
| 1.475 | $-COOCH_3$ | 6–$CH_3$ | $-N(CH_3)_2$ | S | |
| 1.476 | $-COO-C_3H_7$–i | 6–$CH_3$ | $CH_3$ | S | |
| 1.477 | $-COO-C_3H_7$–i | 6–$CH_3$ | $OCH_3$ | S | |
| 1.478 | $-COO-C_3H_7$–i | 6–$CH_3$ | $OCHF_2$ | S | |
| 1.479 | $-COO-C_3H_7$–i | 6–$CH_3$ | $-N(CH_3)_2$ | S | |
| 1.480 | $-COOCH_3$ | 4–$NO_2$ | $CH_3$ | O | 186–187 °C |
| 1.481 | $-COOCH_3$ | 4–$NO_2$ | $OCH_3$ | O | |
| 1.482 | $-COOCH_3$ | 4–$NO_2$ | $OCHF_2$ | O | |
| 1.483 | $-COOCH_3$ | 4–$NO_2$ | $-N(CH_3)_2$ | O | |
| 1.484 | $-COOCH_3$ | 4–$NH_2$ | $CH_3$ | O | |
| 1.485 | $-COOCH_3$ | 4–$NH_2$ | $OCH_3$ | O | |
| 1.486 | $-COOCH_3$ | 4–$NH_2$ | $OCHF_2$ | O | |
| 1.487 | $-COOCH_3$ | 4–$NH_2$ | $-N(CH_3)_2$ | O | |
| 1.488 | Cl | H | $OCH_3$ | S | 165–166 °C |
| 1.489 | Cl | H | $-N(CH_3)_2$ | S | 191 °C (Zers.) |
| 1.490 | $-COO-C_3H_7$–i | H | Cl | O | 163–166 °C |
| 1.491 | $-COO-C_4H_9$–S | H | Cl | O | 165–167 °C |

Tabelle 2:

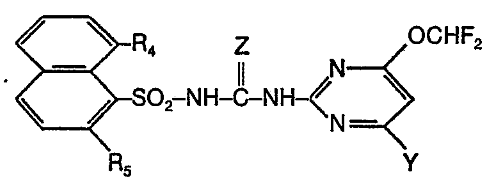

| Nr. | $R_4$ | $R_5$ | Y | Z | Smp. |
|---|---|---|---|---|---|
| 2.1 | H | H | $C_2H_5$ | O | |
| 2.2 | H | H | $OCH_3$ | O | |
| 2.3 | H | H | $-N(CH_3)_2$ | O | |
| 2.4 | H | Cl | $CH_3$ | O | 173–174 °C |
| 2.5 | H | Cl | $-OCHF_2$ | O | |
| 2.6 | H | Cl | $OCH_3$ | O | |
| 2.7 | H | Cl | $C_2H_5$ | O | |
| 2.8 | H | H | $CH_3$ | O | |
| 2.9 | H | H | $-OCHF_2$ | O | |

Tabelle 2: (Fortsetzung)

| Nr. | $R_4$ | $R_5$ | Y | Z | Smp. |
|---|---|---|---|---|---|
| 2.10 | $NO_2$ | H | $CH_3$ | O | |
| 2.11 | $NO_2$ | H | $-OCHF_2$ | O | |
| 2.12 | $NO_2$ | H | $-N(CH_3)_2$ | O | |
| 2.13 | H | $-COOCH_3$ | $-OCHF_2$ | O | |
| 2.14 | H | $-COOCH_3$ | $CH_3$ | O | |
| 2.15 | H | $-SO_2-CH_3$ | $CH_3$ | O | |
| 2.16 | H | $-SO_2-CH_3$ | $-OCHF_2$ | O | |
| 2.17 | H | $CH_3$ | $CH_3$ | O | |
| 2.18 | H | Cl | $-N(CH_3)_2$ | O | |
| 2.19 | $NO_2$ | H | $OCH_3$ | O | |
| 2.20 | H | $-COOCH_3$ | $OCH_3$ | O | |
| 2.21 | H | $-COOCH_3$ | $-N(CH_3)_2$ | O | |
| 2.22 | H | $-SO_2-CH_3$ | $OCH_3$ | O | |
| 2.23 | H | $-SO_2-CH_3$ | $-N(CH_3)_2$ | O | |
| 2.24 | H | $CH_3$ | $OCH_3$ | O | |
| 2.25 | H | $CH_3$ | $OCHF_2$ | O | |
| 2.26 | H | $CH_3$ | $-N(CH_3)_2$ | O | |
| 2.27 | H | $OCH_3$ | $CH_3$ | O | |
| 2.28 | H | $OCH_3$ | $OCH_3$ | O | |
| 2.29 | H | $OCH_3$ | $OCHF_2$ | O | |
| 2.30 | H | $OCH_3$ | $-N(CH_3)_2$ | O | |

Tabelle 3:

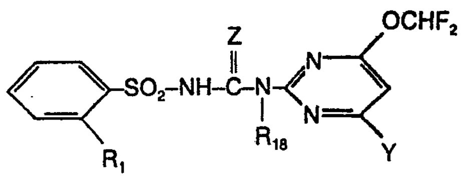

| Nr. | $R_1$ | $R_{18}$ | Y | Z | Smp. |
|---|---|---|---|---|---|
| 3.1 | $-COOCH_3$ | $CH_3$ | $OCHF_2$ | O | 114–115 °C |
| 3.2 | $-COOCH_3$ | $CH_3$ | $CH_3$ | O | |
| 3.3 | $-COOCH_3$ | $CH_3$ | $OCH_3$ | O | |
| 3.4 | $-COOCH_3$ | $CH_3$ | $-N(CH_3)_2$ | O | |
| 3.5 | Cl | $CH_3$ | $CH_3$ | O | |
| 3.6 | Cl | $CH_3$ | $OCHF_2$ | O | |
| 3.7 | $NO_2$ | $CH_3$ | $OCHF_2$ | O | |
| 3.8 | $NO_2$ | $CH_3$ | $CH_3$ | O | |
| 3.9 | $-SO_2-CH_3$ | $CH_3$ | $CH_3$ | O | |
| 3.10 | $-SO_2-CH_3$ | $CH_3$ | $OCHF_2$ | O | |
| 3.11 | $-SO_2-N(CH_3)_2$ | $CH_3$ | $CH_3$ | O | |
| 3.12 | $OCH_3$ | $CH_3$ | $OCHF_2$ | O | |
| 3.13 | $OCH_3$ | $CH_3$ | $CH_3$ | O | |
| 3.14 | $SCH_3$ | $CH_3$ | $CH_3$ | O | |
| 3.15 | $SCH_3$ | $CH_3$ | $OCHF_2$ | O | |
| 3.16 | $NO_2$ | $CH_3$ | $OCHF_2$ | S | |
| 3.17 | $NO_2$ | $CH_3$ | $CH_3$ | S | |

Tabelle 4:

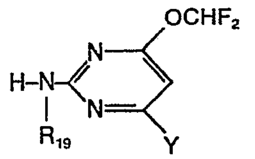

| Nr. | $R_{19}$ | Y | Smp. |
|---|---|---|---|
| 4.1 | $CH_3$ | $OCHF_2$ | 54–55 °C |
| 4.2 | $CH_3$ | Cl | |
| 4.3 | $CH_3$ | $OCH_3$ | |
| 4.4 | $CH_3$ | $-N(CH_3)_2$ | |
| 4.5 | $CH_3$ | $CH_3$ | |
| 4.6 | $CH_3$ | $CF_3$ | |
| 4.7 | $CH_3$ | $CH_2F$ | |
| 4.8 | $OCH_3$ | $CH_3$ | |
| 4.9 | $C_2H_5$ | $OCHF_2$ | |
| 4.10 | $C_2H_5$ | Cl | |
| 4.11 | $C_2H_5$ | $OCH_3$ | |
| 4.12 | $C_2H_5$ | $-N(CH_3)_2$ | |
| 4.13 | $C_2H_5$ | $CH_3$ | |
| 4.14 | $C_3H_7$–n | $CH_3$ | |

Tabelle 5:

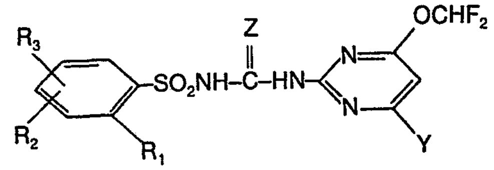

| Nr. | $R_1$ | $R_2$ | $R_3$ | Y | Z | Smp. |
|---|---|---|---|---|---|---|
| 5.1 | $OCH_3$ | 3–Cl | 5–Cl | $CH_3$ | O | 152–155 °C |
| 5.2 | $OCH_3$ | 3–Cl | 5–Cl | $OCH_3$ | O | |
| 5.3 | $OCH_3$ | 3–Cl | 5–Cl | $OCHF_2$ | O | |
| 5.4 | $OCH_3$ | 3–Cl | 5–Cl | Cl | O | |
| 5.5 | $OCH_3$ | 3–Cl | 5–Cl | $-N(CH_3)_2$ | O | |
| 5.6 | $CH_3$ | 3–$CH_3$ | 5–$NO_2$ | $CH_3$ | O | 201 °C (Zers.) |
| 5.7 | $CH_3$ | 3–$CH_3$ | 5–$NO_2$ | $OCH_3$ | O | |
| 5.8 | $CH_3$ | 3–$CH_3$ | 5–$NO_2$ | $OCHF_2$ | O | |
| 5.9 | $CH_3$ | 3–$CH_3$ | 5–$NO_2$ | $-N(CH_3)_2$ | O | |
| 5.10 | $OCH_3$ | 3–Cl | 5–Cl | $CH_3$ | S | |
| 5.11 | $CH_3$ | 3–$CH_3$ | 5–$NO_2$ | $OCH_3$ | S | |

Formulierungsbeispiele
Beispiel 4:
Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5 % |
| Na-Laurylsulfat | 3% | – | – |
| Na-Diisobutyl-naphthalinsulfonat | – | 6% | 6 % |
| Octylphenolpoly-äthylenglykoläther (7–8 Mol AeO) | – | 2% | 2 % |
| Hochdisperse Kieselsäure | 5% | 27% | 27 % |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10% | 1% |
| Octylphenolpolyäthylen-glykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Äthylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37%ige wässrige Formaldehyd-Lösung | 0,2% | 0,2% |
| Silikonöl in Form einer 75%igen wässrigen Emulsion | 0,8% | 0,8% |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel 5:

Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht absorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet:

1: Pflanze nicht gekeimt oder total abgestorben
2–3: sehr starke Wirkung
4–6: mittlere Wirkung
7–8: schwache Wirkung
9: keine Wirkung (wie unbehandelte Kontrolle).

Pre-emergente Wirkung: Konzentration der Wirkstoffemulsion: 70,8 ppm

| Testpflanze Wirkstoff Nr. | Nasturtium | Stellaria | Agrostis | Digitaria |
|---|---|---|---|---|
| 1.1 | 2 | 2 | 1 | 1 |
| 1.3 | 1 | 2 | 1 | 2 |
| 1.4 | 1 | 1 | 1 | 1 |
| 1.7 | 2 | 2 | 1 | 2 |
| 1.10 | 2 | 2 | 1 | 1 |
| 1.11 | 1 | 7 | 1 | 9 |
| 1.12 | 2 | 2 | 2 | 2 |
| 1.13 | 1 | 2 | 1 | 2 |
| 1.16 | 1 | 2 | 1 | 2 |
| 1.18 | 2 | 1 | 1 | 1 |
| 1.19 | 1 | 1 | 1 | 1 |
| 1.20 | 1 | 1 | 1 | 1 |
| 1.21 | 1 | 3 | 1 | 1 |
| 1.33 | 1 | 8 | 1 | 8 |
| 1.42 | 1 | 2 | 1 | 2 |
| 1.43 | 1 | 1 | 1 | 1 |
| 1.67 | 1 | 3 | 1 | 2 |
| 1.78 | 2 | 2 | 1 | 2 |
| 1.79 | 3 | 3 | 5 | 2 |
| 1.83 | 1 | 2 | 1 | 2 |
| 1.85 | 1 | 3 | 1 | 2 |
| 1.93 | 1 | 2 | 1 | 2 |
| 1.94 | 1 | 1 | 1 | 1 |
| 1.100 | 2 | 2 | 2 | 2 |

| Testpflanze Wirkstoff Nr. | Nasturtium | Stellaria | Agrostis | Digitaria |
|---|---|---|---|---|
| 1.101 | 2 | 3 | 1 | 3 |
| 1.102 | 1 | 2 | 1 | 2 |
| 1.109 | 1 | 1 | 1 | 1 |
| 1.110 | 2 | 2 | 2 | 2 |
| 1.113 | 2 | 4 | 2 | 3 |
| 1.114 | 2 | 5 | 3 | 7 |
| 1.130 | 1 | 1 | 1 | 1 |
| 1.139 | 2 | 5 | 2 | 7 |
| 1.140 | 2 | 6 | 2 | 1 |
| 1.144 | 3 | 7 | 3 | 3 |
| 1.146 | 1 | 1 | 1 | 1 |
| 1.148 | 2 | 3 | 1 | 3 |
| 1.149 | 1 | 1 | 2 | 2 |
| 1.310 | 1 | 2 | 1 | 2 |
| 1.317 | 1 | 2 | 1 | 1 |
| 1.342 | 2 | 9 | 2 | 8 |
| 1.352 | 2 | 3 | 2 | 3 |
| 1.375 | 1 | 1 | 1 | 1 |
| 1.381 | 1 | 1 | 1 | 1 |
| 1.387 | 3 | 6 | 3 | 6 |
| 1.390 | 1 | 2 | 1 | 2 |
| 1.395 | 2 | 2 | 1 | 4 |
| 1.401 | 1 | 6 | 1 | 6 |
| 2.4 | 2 | 8 | 2 | 8 |
| 3.1 | 1 | 1 | 1 | 1 |
| 5.1 | 2 | 7 | 3 | 9 |

Beispiel 6:
Nachweis der Selektivität bei Vorauflaufanwendung

Im Gewächshaus werden Pflanzensamen von dikotylen und monokotylen Unkräutern und Kulturpflanzen in Töpfe von 11 cm Durchmesser gesät. Unmittelbar danach wird die Erdoberfläche mit einer wässrigen Dispersion oder Lösung der Wirkstoffe behandelt. Es werden Konzentrationen von 0,250, 0,125 und 0,06 kg Wirkstoffmengen pro Hektar angewendet. Die Töpfe werden dann im Gewächshaus bei einer Temperatur von 22–25 °C und 50–70% relativer Luftfeuchtigkeit gehalten. Nach 3 Wochen wird der Versuch ausgewertet und die Wirkung nach dem gleichen Massstab, wie im Versuch 5 angegeben, beurteilt.

Versuchsergebnisse (pre-emergent)

| Wirkung Aufwandmenge kg AS/ha | Verb. Nr. 1.1 | | | Verb. Nr. 1.2 | | | Verb. Nr. 1.5 | | | Verb. Nr. 1.18 | | |
|---|---|---|---|---|---|---|---|---|---|---|---|---|
| Testpflanze | 0.25 | 0.12 | 0.06 | 0.25 | 0.12 | 0.06 | 0.25 | 0.12 | 0.06 | 0.25 | 0.12 | 0.06 |
| Mais | 2 | 3 | 4 | 8 | 9 | 9 | 4 | 6 | 7 | 3 | 3 | 5 |
| Avana fatua | 2 | 2 | 2 | 2 | 2 | 2 | 3 | 4 | 5 | 3 | 4 | 7 |
| Alopecurus myos. | 2 | 2 | 2 | 2 | 2 | 2 | 2 | 2 | 3 | 3 | 3 | 4 |
| Echinochloa c.g. | 2 | 2 | 3 | 4 | 4 | 4 | 2 | 2 | 4 | 2 | 2 | 2 |
| Cyperus escul. | 1 | 1 | 1 | 1 | 2 | 2 | 2 | 3 | 4 | 3 | 3 | 4 |
| Soja | 6 | 7 | 9 | 4 | 5 | 6 | 7 | 9 | 9 | 7 | 7 | 7 |
| Abutilon | 1 | 1 | 3 | 1 | 1 | 1 | 3 | 3 | 3 | 2 | 2 | 3 |
| Xanthium Sp. | 2 | 3 | 4 | 1 | 1 | 2 | 4 | 4 | 8 | 4 | 4 | 4 |
| Chenopodium Sp. | 3 | 3 | 3 | 1 | 2 | 3 | 4 | 4 | 4 | 2 | 2 | 3 |
| Sinapis | 2 | 2 | 2 | 1 | 1 | 1 | 2 | 2 | 2 | 3 | 4 | 4 |
| Stellaria | 3 | 3 | 3 | 2 | 2 | 2 | 3 | 3 | 3 | – | – | – |
| Chrysanthe. leuc. | 2 | 2 | 2 | 2 | 2 | 2 | 2 | 3 | 3 | – | – | – |
| Galium aparine | 2 | 3 | 3 | 2 | 2 | 2 | 2 | 3 | 3 | 3 | 3 | 3 |
| Viola tricolor | 2 | 2 | 2 | 2 | 2 | 2 | 3 | 3 | 3 | 2 | 2 | 2 |
| Veronica Sp. | 2 | 2 | 2 | 1 | 1 | 1 | 1 | 1 | 1 | – | – | – |

– : nicht geprüft.

Beispiel 7:
Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blattstadium mit einer wässrigen Wirkstoffdispersion in Dosierungen von 0,5 kg AS/ha gespritzt und dann bei 24° bis 26 °C und 45–60% relativer Luftfeuchtigkeit gehalten. 15 Tage nach der Behandlung wird der Versuch ausgewertet und nach dem gleichen Massstab wie im pre-emergenten Versuch bewertet.

post-emergente Wirkung: Aufwandmenge: 0,5 kg Wirksubstanz/Hektar

| Verb. Nr. | Avena | Setaria | Lolium | Solanum | Sinapis | Stellaria | Phaseolus |
|---|---|---|---|---|---|---|---|
| 1.1 | 3 | 4 | 4 | 4 | 3 | 3 | 7 |
| 1.2 | 3 | 3 | 4 | 3 | 3 | 2 | 3 |
| 1.5 | 5 | 4 | 5 | 3 | 4 | 4 | 7 |

Beispiel 8:
Nachweis der Wuchshemmung bei tropischen Bodendeckern-Leguminosen (cover crops)

Die Versuchspflanzen (psophocarpus palustris und centrosema pubescens) werden bis zum ausgewachsenen Stadium herangezogen und bis auf eine Höhe von 15 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen).

Beispiel 9:
Wuchsregulierung an Sojabohnen

In Kunststoffbehältern mit einem Erde-Torf-Sandgemisch im Verhältnis 6:3:1 werden Sojabohnen der Sorte «Hark» angesät und in eine Klimakammer gegeben. Durch optimale Temperaturwahl, Beleuchtung, Düngerzugabe und Bewässerung entwickeln sich die Pflanzen nach ca. 5 Wochen bis zum 5–6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

| Verb. Nr. | Aufwandmenge g/ha | Schoten am Haupttrieb in % zur Kontrolle Anzahl | Gewicht |
|---|---|---|---|
| 1.1 | 3 | 110 | 112 |
| | 10 | 120 | 118 |
| | 30 | 110 | 112 |
| Kontrolle | 0 | 100 | 100 |

Beispiel 10:
Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine deutliche Verringerung des Neuzuwachses, sowie teilweise eine Zunahme der Stengeldurchmesser auf.

| Verb. Nr. | Neuzuwachs (Wuchshöhe) in % zur Kontrolle Gerste 50 g AS/ha | Roggen 50 g AS/ha |
|---|---|---|
| 1.1 | – | 60 |
| 1.2 | 0 | – |
| 1.3 | 100 | 85 |
| 1.4 | 70 | 85 |
| 1.5 | 60 | – |
| 1.18 | 50 | 70 |
| 1.102 | 15 | 90 |
| Kontrolle | 100 | 100 |

– : nicht geprüft

Beispiel 11:
Wuchshemmung bei Gräsern

In Kunststoffschalen mit Erde-Torf-Sand-Gemisch (6:3:1) werden die Gräser Lolium perenne, Poy pratensis, Festuca ovina, Dactylis glomerata und Cynodon dactylon als Gemisch im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt. Die Verbindungen der Formel I bewirken eine deutliche Reduzierung des Neuzuwachses im Vergleich zur unbehandelten Kontrolle.

| Verb. Nr. | Neuzuwachs (Wuchshöhe) in % zur Kontrolle Grasmischung 50 g AS/ha |
|---|---|
| 1.1 | 16 |
| 1.2 | 15 |
| 1.3 | 65 |

| Verb. Nr. | Neuzuwachs (Wuchshöhe) in % zur Kontrolle |
|---|---|
| | Grasmischung 50 g AS/ha |
| 1.4 | 15 |
| 1.5 | 60 |
| 1.18 | 40 |
| 1.102 | 10 |
| Kontrolle | 100 |

**Patentansprüche für die Vertragsstaaten BE, CH, DE, FR, GB, IT, LI, NL, SE**

1. N-Arylsulfonyl-N'-pyrimidinylharnstoffe der allgemeinen Formel I

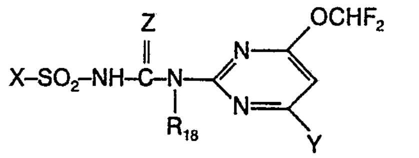
(I)

worin

X einen Rest der Formel

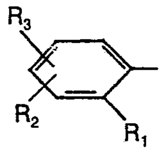 oder 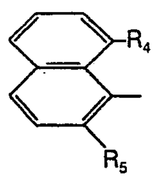 ,

Y $C_1$–$C_3$-Alkyl, $C_1$–$C_3$-Halogenalkyl, $C_1$–$C_3$-Alkoxy, $C_1$–$C_3$-Halogenalkoxy, $C_2$–$C_3$-Alkoxyalkyl, $C_1$–$C_3$-Alkylthio, Halogen oder $-NR_{16}R_{17}$,

Z Sauerstoff oder Schwefel,

$R_1$ Wasserstoff, Halogen, Cyan, Nitro, $C_1$–$C_4$-Halogenalkyl, $C_1$–$C_4$-Alkyl, $C_1$–$C_4$-Alkoxy, $-CO-R_6$, $-NR_7R_8$, $-S(O)_m-C_1-C_4$-Alkyl, oder $-SO_2R_9$,

$R_2$ Wasserstoff, Fluor, Chlor, Brom, Nitro, Trifluormethyl, $-NR_{20}R_{21}$, Methyl, Äthyl, Methoxy, Äthoxy oder $-S(O)_m-C_1-C_4$-Alkyl,

$R_3$ Wasserstoff, Fluor, Chlor, Brom, Amino, Nitro oder Methoxy,

$R_6$ Wasserstoff, $C_1$–$C_4$-Alkyl, $C_3$–$C_5$-Alkenyloxy, $C_3$–$C_5$-Alkinyloxy, $C_1$–$C_4$-Halogenalkyl, $C_1$–$C_5$-Alkylthio, Phenoxy, Benzyloxy, $-NR_{10}R_{11}$ oder gegebenenfalls durch 1–3 Halogenatome oder $C_1$–$C_3$-Alkoxy substituiertes $C_1$–$C_5$-Alkoxy,

$R_7$ Wasserstoff, Methoxy, Äthoxy, $C_1$–$C_4$-Alkyl oder $-CO-R_{12}$,

$R_8$ Wasserstoff oder $C_1$–$C_4$-Alkyl,

$R_9$ eine Gruppe $-O-R_{13}$ oder $-NR_{14}R_{15}$,

$R_{13}$ gegebenenfalls durch 1–3 Halogenatome substituiertes $C_1$–$C_4$-Alkyl, Phenyl oder Benzyl,

$R_{18}$ Wasserstoff, $C_1$–$C_3$-Alkyl oder $C_1$–$C_3$-Alkoxy und

m die Zahlen Null, eins oder zwei bedeuten, wobei $R_4$ die gleiche Bedeutung wie $R_2$; $R_5$ die gleiche Bedeutung wie $R_1$; $R_{10}$, $R_{11}$, $R_{14}$ und $R_{20}$ die gleiche Bedeutung wie $R_7$; und $R_{12}$, $R_{15}$, $R_{16}$, $R_{17}$ und $R_{21}$ die gleiche Bedeutung wie $R_8$ haben, sowie die Salze dieser Verbindungen.

2. N-Arylsulfonyl-N'-pyrimidinylharnstoffe der allgemeinen Formel Ia

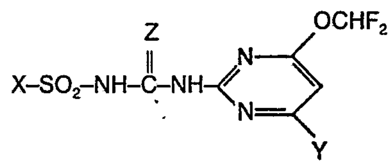
(Ia)

entsprechen, worin

X einen Rest der Formel

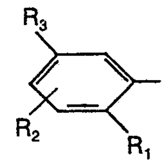 oder 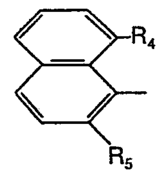 ,

Y Methyl, Äthyl, Methoxy, Äthoxy, Difluormethoxy, Dimethylamino oder Äthylmethylamino,

Z Sauerstoff oder Schwefel,

$R_1$ Wasserstoff, Fluor, Chlor, Brom, Jod, Cyan, Nitro, Trifluormethyl, $C_1$–$C_4$-Alkyl, $C_1$–$C_4$-Alkoxy, $-CO-R_6$, $-NR_7R_8$, $-S(O)_m-C_1-C_4$-Alkyl oder $-SO_2-R_9$,

$R_2$ Wasserstoff, Fluor, Chlor, Brom, Nitro, Trifluormethyl, Amino, Methyl, Äthyl, Methoxy, Äthoxy oder $-S(O)_m-C_1-C_3$-Alkyl,

$R_3$ Wasserstoff, Fluor, Chlor, Brom, Nitro oder Methoxy,

$R_6$ Wasserstoff, $C_1$–$C_3$-Alkyl, $C_3$–$C_5$-Alkenyloxy, $C_3$–$C_5$-Alkinyloxy, $C_1$–$C_5$-Alkylthio, Phenoxy, Benzyloxy, $-NR_{10}R_{11}$ oder gegebenenfalls durch 1–3 Halogenatome substituiertes $C_1$–$C_5$-Alkoxy,

$R_7$ Wasserstoff, Methoxy, Äthoxy, $C_1$–$C_4$-Alkyl oder $-CO-R_{12}$,

$R_8$ Wasserstoff oder $C_1$–$C_4$-Alkyl,

$R_9$ eine Gruppe $-O-R_{13}$ oder $-NR_{14}R_{15}$,

$R_{13}$ $C_1$–$C_4$-Alkyl, Phenyl oder Benzyl und

m die Zahlen Null, eins oder zwei bedeuten, wobei $R_4$ die gleiche Bedeutung wie $R_2$; $R_5$ die gleiche Bedeutung wie $R_1$; $R_{10}$ und $R_{14}$ die gleiche Bedeutung wie $R_7$ und $R_{11}$, $R_{12}$ und $R_{15}$ die gleiche Bedeutung wie $R_8$ haben, sowie den Salzen dieser Verbindungen.

3. Verbindungen gemäss Anspruch 1, dadurch gekennzeichnet, dass X den gegebenenfalls substituierten Phenylrest bedeutet.

4. Verbindungen gemäss Anspruch 1, dadurch gekennzeichnet, dass Y einen Rest mit höchstens 2 Kohlenstoffatomen bedeutet.

5. Verbindungen gemäss Anspruch 1, dadurch gekennzeichnet, dass Z Sauerstoff bedeutet.

6. Verbindungen gemäss Anspruch 1, dadurch gekennzeichnet, dass $R_3$ und $R_4$ Wasserstoff bedeuten.

7. Verbindungen gemäss Anspruch 1, dadurch gekennzeichnet, dass $R_{18}$ Wasserstoff oder Methyl bedeutet.

8. Verbindungen gemäss Anspruch 1, dadurch gekennzeichnet, dass X den gegebenenfalls substituierten Phenylrest, Y einen Rest mit höchstens 2 Kohlenstoffatomen, Z Sauerstoff und $R_3$ und $R_{18}$ Wasserstoff bedeuten.

9. Verbindungen gemäss Anspruch 8, dadurch gekennzeichnet, dass $R_2$ Wasserstoff bedeutet.

10. Verbindungen gemäss Anspruch 1, dadurch gekennzeichnet, dass X den gegebenenfalls substituierten Phenylkern, Y einen Rest mit höchstens 2 Kohlenstoffatomen, Z Sauerstoff und $R_{18}$ Wasserstoff bedeuten, wobei

$R_1$ für Wasserstoff, Halogen, Nitro, $C_1$–$C_4$-Halogenalkyl, $C_1$–$C_4$-Alkoxy, $C_1$–$C_4$-Alkyl, $-S(O)_m-C_1-C_4$-Alkyl, $-SO_2-N(CH_3)_2$, $-SO_2-OCH_2CF_3$ oder $-CO-R_6$,

$R_2$ für Wasserstoff, Fluor, Chlor, Nitro, Amino, Trifluormethyl, Methyl, Methoxy, Äthoxy oder $-S(O)_m-C_1-C_4$-Alkyl,

$R_6$ für Wasserstoff, Methyl, $C_3$–$C_5$-Alkenyloxy, $C_3$–$C_5$-Alkinyl, $C_1$–$C_3$-Alkylthio, Dimethylamino, Methylamino, Amino oder gegebenenfalls durch 1 bis 3 Halogenatome oder $C_1$–$C_3$-Alkoxy substituiertes $C_1$–$C_5$-Alkoxy und

m für die Zahlen Null, eins oder zwei stehen.

11. N-(2-Chlorphenyl-sulfonyl)-N′-(4-difluormethoxy-6-methyl-pyrimidin-2-yl)-harnstoff gemäss Anspruch 1.

12. N-(2-Methoxycarbonylphenyl-sulfonyl)-N′-[4,6-bis-(difluormethoxy)-pyrimidin-2-yl]-harnstoff gemäss Anspruch 1.

13. N-(2-Methoxycarbonylphenyl-sulfonyl)-N′-(4-difluormethoxy-6-methylpyrimidin-2-yl)-harnstoff gemäss Anspruch 1.

14. N-(2-Methoxycarbonylphenyl-sulfonyl)-N′-(4-difluormethoxy-6-methoxy-pyrimidin-2-yl)-harnstoff gemäss Anspruch 1.

15. N-(2-Nitrophenyl-sulfonyl)-N′-(4-difluormethoxy-6-methyl-pyrimidin-2-yl)-harnstoff gemäss Anspruch 1.

16. Aminopyrimidine der Formel Va

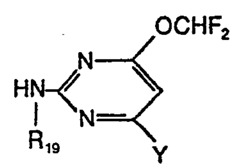
(Va)

worin Y die unter Formel I, in Anspruch 1, gegebene Bedeutung hat und $R_{19}$ für $C_1$–$C_3$-Alkyl oder $C_1$–$C_3$-Alkoxy steht.

17. Verfahren zur Herstellung der Verbindungen der Formel I, gemäss Anspruch 1, dadurch gekennzeichnet, dass man ein Arylsulfonamid der Formel II

$$X-SO_2-NH_2 \quad (II),$$

worin X die unter Formel I gegebene Bedeutung hat, in Gegenwart einer Base mit einem N-Pyrimidinylcarbamat der Formel III

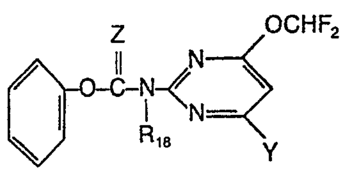
(III),

worin $R_{18}$, Y und Z die unter Formel I gegebene Bedeutung haben, umsetzt und gegebenenfalls in die Salze überführt.

18. Verfahren zur Herstellung der Verbindungen der Formel I, gemäss Anspruch 1, dadurch gekennzeichnet, dass man ein Arylsulfonylisocyanat oder -isothiocyanat der Formel IV

$$X-SO_2-N=C=Z \quad (IV)$$

worin X und Z die unter Formel I gegebene Bedeutung haben, gegebenenfalls in Gegenwart einer Base, mit einem Aminopyrimidin der Formel V

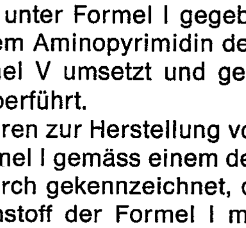
(V),

worin $R_{18}$ und Y die unter Formel I gegebene Bedeutung haben, umsetzt und gegebenenfalls in die Salze überführt.

19. Verfahren zur Herstellung der Verbindungen der Formel I, gemäss Anspruch 1, worin $R_{18}$ Wasserstoff bedeutet, dadurch gekennzeichnet, dass man ein Arylsulfonamid der oben angegebenen Formel II gegebenenfalls in Gegenwart einer Base mit einem Isocyanat oder Isothiocyanat der Formel VI

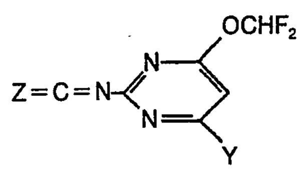
(VI),

worin Y und Z die unter Formel I gegebene Bedeutung haben, umsetzt und gegebenenfalls in die Salze überführt.

20. Verfahren zur Herstellung der Verbindungen der Formel I gemäss Anspruch 1, dadurch gekennzeichnet, dass man ein N-Arylsulfonylcarbamat der Formel VII

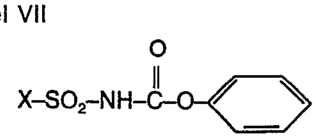
(VII),

worin X die unter Formel I gegebene Bedeutung hat, mit einem Aminopyrimidin der oben angegebenen Formel V umsetzt und gegebenenfalls in die Salze überführt.

21. Verfahren zur Herstellung von Additionssalzen der Formel I gemäss einem der Ansprüche 17 bis 20, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

22. Ein herbizides und den Pflanzenwuchs regulierendes Mittel, dadurch gekennzeichnet, dass es neben Träger- und/oder anderen Zuschlagstoffen als Wirkstoff mindestens einen N-Arylsulfonyl-N′-pyrimidinyl-harnstoff der Formel I, Anspruch 1, enthält.

23. Die Verwendung der N-Arylsulfonyl-N′-pyrimidinyl-harnstoffe der Formel I, Anspruch 1, oder

sie enthaltender Mittel zur Bekämpfung unerwünschten Pflanzenwachstums.

24. Die Verwendung der N-Arylsulfonyl-N'-pyrimidinyl-harnstoffe der Formel I, Anspruch 1, oder sie enthaltender Mittel zur Regulierung des Pflanzenwachstums.

25. Die Verwendung der N-Phenylsulfonyl-N'-triazinyl- oder -pyrimidinylharnstoffe der Formel I, Anspruch 1, oder sie enthaltender Mittel zur Wuchsregulierung von Kulturpflanzen zum Zwekke einer Ertragssteigerung.

26. Die Verwendung gemäss Anspruch 23 zur selektiven pre- oder postemergenten Bekämpfung von Unkräutern in Nutzpflanzenkulturen.

27. Die Verwendung gemäss Anspruch 26 in Zuckerrohr-, Mais- und Baumwollkulturen.

28. Die Verwendung gemäss Anspruch 26 in Sojakulturen.

29. Die Verwendung gemäss Anspruch 26 zur Bekämpfung perennierender Unkräuter in Nutzpflanzenkulturen.

30. Die Verwendung gemäss Anspruch 24 zur Unterdrückung des Pflanzenwachstums über das 2-Blattstadium hinaus, dadurch gekennzeichnet, dass die Wirkstoffe preemergent angewendet werden.

31. Die Verwendung gemäss Anspruch 25 in Sojakulturen.

32. Die Verwendung gemäss Anspruch 24 bei Bodendecker-Leguminosen.

**Patentansprüche für den Vertragsstaat AT.**

1. Ein herbizides und den Pflanzenwuchs regulierendes Mittel, dadurch gekennzeichnet, dass es neben Träger- und/oder anderen Zuschlagstoffen als Wirkstoff mindestens einen N-Arylsulfonyl-N'-pyrimidinyl-harnstoff der Formel I

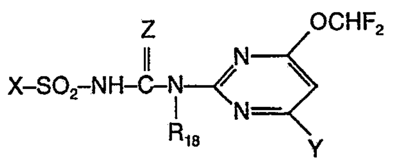

(I)

enthält, worin

X einen Rest der Formel

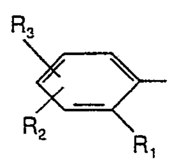

oder

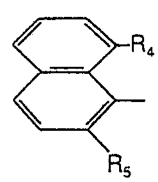

,

Y $C_1$–$C_3$-Alkyl, $C_1$–$C_3$-Halogenalkyl, $C_1$–$C_3$-Alkoxy, $C_1$–$C_3$-Halogenalkoxy, $C_2$–$C_3$-Alkoxyalkyl, $C_1$–$C_3$-Alkylthio, Halogen oder $-NR_{16}R_{17}$,

Z Sauerstoff oder Schwefel,

$R_1$ Wasserstoff, Halogen, Cyan, Nitro, $C_1$–$C_4$-Halogenalkyl, $C_1$–$C_4$-Alkyl, $C_1$–$C_4$-Alkoxy, $-CO-R_6$, $-NR_7R_8$, $-S(O)_m-C_1$–$C_4$-Alkyl, oder $-SO_2R_9$,

$R_2$ Wasserstoff, Fluor, Chlor, Brom, Nitro, Trifluormethyl, $-NR_{20}R_{21}$, Methyl, Äthyl, Methoxy, Äthoxy oder $-S(O)_m-C_1$–$C_4$-Alkyl,

$R_3$ Wasserstoff, Fluor, Chlor, Brom, Amino, Nitro oder Methoxy,

$R_6$ Wasserstoff, $C_1$–$C_4$-Alkyl, $C_3$–$C_5$-Alkenyloxy, $C_3$–$C_5$-Alkinyloxy, $C_1$–$C_4$-Halogenalkyl, $C_1$–$C_5$-Alkylthio, Phenoxy, Benzyloxy, $-NR_{10}R_{11}$ oder gegebenenfalls durch 1–3 Halogenatome oder $C_1$–$C_3$-Alkoxy substituiertes $C_1$–$C_5$-Alkoxy,

$R_7$ Wasserstoff, Methoxy, Äthoxy, $C_1$–$C_4$-Alkyl oder $-CO-R_{12}$,

$R_8$ Wasserstoff oder $C_1$–$C_4$-Alkyl,

$R_9$ eine Gruppe $-O-R_{13}$ oder $-NR_{14}R_{15}$,

$R_{13}$ gegebenenfalls durch 1–3 Halogenatome substituiertes $C_1$–$C_4$-Alkyl, Phenyl oder Benzyl,

$R_{18}$ Wasserstoff, $C_1$–$C_3$-Alkyl oder $C_1$–$C_3$-Alkoxy und

m die Zahlen Null, eins oder zwei bedeuten, wobei $R_4$ die gleiche Bedeutung wie $R_2$; $R_5$ die gleiche Bedeutung wie $R_1$; $R_{10}$, $R_{11}$, $R_{14}$ und $R_{20}$ die gleiche Bedeutung wie $R_7$; und $R_{12}$, $R_{15}$, $R_{16}$, $R_{17}$ und $R_{21}$ die gleiche Bedeutung wie $R_8$ haben, sowie die Salze dieser Verbindungen.

2. Mittel gemäss Anspruch 1, dadurch gekennzeichnet, dass X den gegebenenfalls substituierten Phenylrest bedeutet.

3. Mittel gemäss Anspruch 1, dadurch gekennzeichnet, dass Y einen Rest mit höchstens 2 Kohlenstoffatomen bedeutet.

4. Mittel gemäss Anspruch 1, dadurch gekennzeichnet, dass Z Sauerstoff bedeutet.

5. Mittel gemäss Anspruch 1, dadurch gekennzeichnet, dass $R_3$ und $R_4$ Wasserstoff bedeuten.

6. Mittel gemäss Anspruch 1, dadurch gekennzeichnet, dass $R_{18}$ Wasserstoff oder Methyl bedeutet.

7. Mittel gemäss Anspruch 1, dadurch gekennzeichnet, dass X den gegebenenfalls substituierten Phenylrest, Y einen Rest mit höchstens 2 Kohlenstoffatomen, Z Sauerstoff und $R_3$ und $R_{18}$ Wasserstoff bedeuten.

8. Mittel gemäss Anspruch 7, dadurch gekennzeichnet, dass $R_2$ Wasserstoff bedeutet.

9. Mittel gemäss Anspruch 1, dadurch gekennzeichnet, dass X den gegebenenfalls substituierten Phenylkern, Y einen Rest mit höchstens 2 Kohlenstoffatomen, Z Sauerstoff und $R_{18}$ Wasserstoff bedeuten, wobei

$R_1$ für Wasserstoff, Halogen, Nitro, $C_1$–$C_4$-Halogenalkyl, $C_1$–$C_4$-Alkoxy, $C_1$–$C_4$-Alkyl, $-S(O)_m-C_1$–$C_4$-Alkyl, $-SO_2-N(CH_3)_2$, $-SO_2-OCH_2CF_3$ oder $-CO-R_6$,

$R_2$ für Wasserstoff, Fluor, Chlor, Nitro, Amino, Trifluormethyl, Methyl, Methoxy, Äthoxy oder $-S(O)_m-C_1$–$C_4$-Alkyl,

$R_6$ für Wasserstoff, Methyl, $C_3$–$C_5$-Alkenyloxy, $C_3$–$C_5$-Alkinyl, $C_1$–$C_3$-Alkylthio, Dimethylamino, Methylamino, Amino oder gegebenenfalls durch 1 bis 3 Halogenatome oder $C_1$–$C_3$-Alkoxy substituiertes $C_1$–$C_5$-Alkoxy und

m für die Zahlen Null, eins oder zwei stehen.

10. N-(2-Chlorphenyl-sulfonyl)-N'-(4-difluormethoxy-6-methyl-pyrimidin-2-yl)-harnstoff gemäss Anspruch 1.

11. N-(2-Methoxycarbonylphenyl-sulfonyl)-N'-[4,6-bis-(difluormethoxy)-pyrimidin-2-yl]-harnstoff gemäss Anspruch 1.

12. N-(2-Methoxycarbonylphenyl-sulfonyl)-N'-(4-difluormethoxy-6-methyl-pyrimidin-2-yl)-harnstoff gemäss Anspruch 1.

13. N-(2-Methoxycarbonylphenyl-sulfonyl)-N'-(4-difluormethoxy-6-methoxy-pyrimidin-2-yl)-harnstoff gemäss Anspruch 1.

14. N-(2-Nitrophenyl-sulfonyl)-N'-(4-difluor-methoxy-6-methyl-pyrimidin-2-yl)-harnstoff gemäss Anspruch 1.

15. Verfahren zur Herstellung der Verbindungen der Formel I, gemäss Anspruch 1, dadurch gekennzeichnet, dass man entweder

a) ein Arylsulfonamid der Formel II

$$X{-}SO_2{-}NH_2 \qquad (II),$$

worin X die unter Formel I gegebene Bedeutung hat, in Gegenwart einer Base mit einem N-Pyrimidinylcarbamat der Formel III

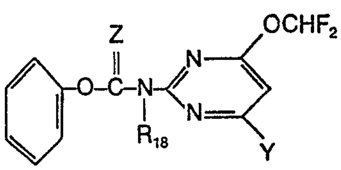

(III),

worin $R_{18}$, Y und Z die unter Formel I gegebene Bedeutung haben, umsetzt oder

b) ein Arylsulfonylisocyanat oder -isothiocyanat der Formel IV

$$X{-}SO_2{-}N{=}C{=}Z \qquad (IV)$$

worin X und Z die unter Formel I gegebene Bedeutung haben, gegebenenfalls in Gegenwart einer Base, mit einem Aminopyrimidin der Formel V

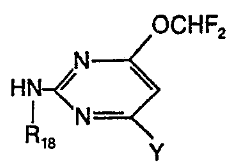

(V),

worin $R_{18}$ und Y die unter Formel I gegebene Bedeutung haben, umsetzt oder

c) ein N-Arylsulfonylcarbamat der Formel VII

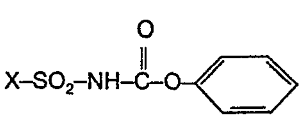

(VII),

worin X die unter Formel I gegebene Bedeutung hat, mit einem Aminopyrimidin der oben angegebenen Formel V umsetzt und gegebenenfalls dadurch in die Salze überführt, dass man einen Sulfonylharnstoff der Formel I, mit einem Amin, einem Alkalimetall- oder Erdalkalimetallhydroxid oder einer quaternären Ammoniumbase umsetzt.

16. Verfahren zur Herstellung der Verbindungen der Formel I, gemäss Anspruch 1, worin $R_{18}$ Wasserstoff bedeutet, dadurch gekennzeichnet, dass man ein Arylsulfonamid der oben angegebenen Formel II gegebenenfalls in Gegenwart einer Base mit einem Isocyanat oder Isothiocyanat der Formel VI

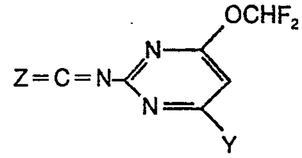

(VI),

worin Y und Z die unter Formel I gegebene Bedeutung haben, umsetzt und gegebenenfalls in die Salze überführt.

17. Die Verwendung der N-Arylsulfonyl-N'-pyrimidinyl-harnstoffe der Formel I, Anspruch 1, oder sie enthaltender Mittel zur Bekämpfung unerwünschten Pflanzenwachstums.

18. Die Verwendung der N-Arylsulfonyl-N'-pyrimidinyl-harnstoffe der Formel I, Anspruch 1, oder sie enthaltender Mittel zur Regulierung des Pflanzenwachstums.

19. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwekke einer Ertragssteigerung.

20. Die Verwendung gemäss Anspruch 17 zur selektiven pre- oder postemergenten Bekämpfung von Unkräutern in Nutzpflanzenkulturen.

21. Die Verwendung gemäss Anspruch 20 in Zuckerrohr-, Mais- und Baumwollkulturen.

22. Die Verwendung gemäss Anspruch 20 in Sojakulturen.

23. Die Verwendung gemäss Anspruch 20 zur Bekämpfung perennierender Unkräuter in Nutzpflanzenkulturen.

24. Die Verwendung gemäss Anspruch 18 zur Unterdrückung des Pflanzenwachstums über das 2-Blattstadium hinaus, dadurch gekennzeichnet, dass die Wirkstoffe preemergent angewendet werden.

25. Die Verwendung gemäss Anspruch 19 in Sojakulturen.

26. Die Verwendung gemäss Anspruch 18 bei Bodendecker-Leguminosen.

**Claims for the contracting states BE, CH, DE, FR, GB, IT, LI, NL, SE.**

1. N-arylsulphonyl-N'-pyrimidinylureas of the general formula I

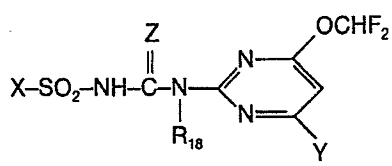

(I)

wherein

X represents a radical of the formula

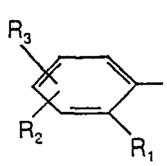

or

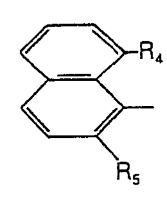

,

Y represents $C_1$–$C_3$ alkyl, $C_1$–$C_3$ haloalkyl, $C_1$–$C_3$ alkoxy, $C_1$–$C_3$ haloalkoxy, $C_2$–$C_3$ alkoxyalkyl, $C_1$–$C_3$ alkylthio, halogen or $-NR_{16}R_{17}$,

Z represents oxygen or sulphur,

$R_1$ represents hydrogen, halogen, cyano, nitro, $C_1$–$C_4$ haloalkyl, $C_1$–$C_4$ alkyl, $C_1$–$C_4$ alkoxy, $-CO-R_6$, $-NR_7R_8$, $-S(O)_m-C_1-C_4$ alkyl or $-SO_2R_9$,

$R_2$ represents hydrogen, fluorine, chlorine, bromine, nitro, trifluoromethyl, $-NR_{20}R_{21}$, methyl, ethyl, methoxy, ethoxy or $-S(O)_m-C_1-C_4$ alkyl,

$R_3$ represents hydrogen, fluorine, chlorine, bromine, amino, nitro or methoxy,

$R_6$ represents hydrogen, $C_1$–$C_4$ alkyl, $C_3$–$C_5$ alkenyloxy, $C_3$–$C_5$ alkynyloxy, $C_1$–$C_4$ haloalkyl, $C_1$–$C_5$ alkylthio, phenoxy, benzyloxy, $-NR_{10}R_{11}$, or $C_1$–$C_5$ alkoxy that is optionally substituted by from 1 to 3 halogen atoms or by $C_1$–$C_3$ alkoxy,

$R_7$ represents hydrogen, methoxy, ethoxy, $C_1$–$C_4$ alkyl or $-CO-R_{12}$,

$R_8$ represents hydrogen or $C_1$–$C_4$ alkyl,

$R_9$ represents an $O-R_{13}$ or $-NR_{14}R_{15}$ group,

$R_{13}$ represents $C_1$–$C_4$ alkyl that is optionally substituted by from 1 to 3 halogen atoms, or represents phenyl or benzyl,

$R_{18}$ represents hydrogen, $C_1$–$C_3$ alkyl or $C_1$–$C_3$ alkoxy, and

m is 0, 1 or 2,

and $R_4$ has the same meaning as $R_2$; $R_5$ has the same meaning as $R_1$; each of $R_{10}$, $R_{11}$, $R_{14}$ and $R_{20}$ has the same meaning as $R_7$; and each of $R_{12}$, $R_{15}$, $R_{16}$, $R_{17}$ and $R_{21}$ has the same meaning as $R_8$, and the salts of those compounds.

2. N-arylsulphonyl-N′-pyrimidinylureas of the general formula Ia

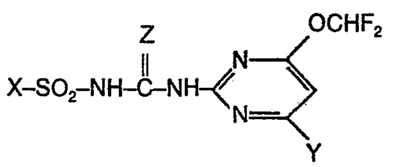 (Ia)

wherein

X represents a radical of the formula

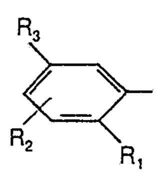 or 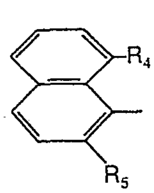 ,

Y represents methyl, ethyl, methoxy, ethoxy, difluoromethoxy, dimethylamino or ethylmethylamino,

Z represents oxygen or sulphur,

$R_1$ represents hydrogen, fluorine, chlorine, bromine, iodine, cyano, nitro, trifluoromethyl, $C_1$–$C_4$ alkyl, $C_1$–$C_4$ alkoxy, $-CO-R_6$, $-NR_7R_8$, $-S(O)_m-C_1-C_4$ alkyl or $-SO_2-R_9$,

$R_2$ represents hydrogen, fluorine, chlorine, bromine, nitro, trifluoromethyl, amino, methyl, ethyl, methoxy, ethoxy or $-S(O)_m-C_1-C_3$ alkyl,

$R_3$ represents hydrogen, fluorine, chlorine, bromine, nitro or methoxy,

$R_6$ represents hydrogen, $C_1$–$C_3$ alkyl, $C_3$–$C_5$ alkenyloxy, $C_3$–$C_5$ alkynyloxy, $C_1$–$C_5$ alkylthio, phenoxy, benzyloxy, $-NR_{10}R_{11}$, or $C_1$–$C_5$ alkoxy that is optionally substituted by from 1 to 3 halogen atoms,

$R_7$ represents hydrogen, methoxy, ethoxy, $C_1$–$C_4$ alkyl or $-CO-R_{12}$,

$R_8$ represents hydrogen or $C_1$–$C_4$ alkyl,

$R_9$ represents an $-O-R_{13}$ or $-NR_{14}R_{15}$ group,

$R_{13}$ represents $C_1$–$C_4$ alkyl, phenyl or benzyl and

m is 0, 1 or 2,

and $R_4$ has the same meaning as $R_2$; $R_5$ has the same meaning as $R_1$; each of $R_{10}$ and $R_{14}$ has the same meaning as $R_7$; and each of $R_{11}$, $R_{12}$ and $R_{15}$ has the same meaning as $R_8$, and the salts of those compounds.

3. Compounds according to claim 1, characterised in that X represents the optionally substituted phenyl radical.

4. Compounds according to claim 1, characterised in that Y represents a radical containing a maximum of 2 carbon atoms.

5. Compounds according to claim 1, characterised in that Z represents oxygen.

6. Compounds according to claim 1, characterised in that $R_3$ and $R_4$ represent hydrogen.

7. Compounds according to claim 1, characterised in that $R_{18}$ represents hydrogen or methyl.

8. Compounds according to claim 1, characterised in that X represents the optionally substituted phenyl radical, Y represents a radical containing a maximum of 2 carbon atoms, Z represents oxygen and $R_3$ and $R_{18}$ represent hydrogen.

9. Compounds according to claim 8, characterised in that $R_2$ represents hydrogen.

10. Compounds according to claim 1, characterised in that X represents the optionally substituted phenyl nucleus, Y represents a radical containing a maximum of 2 carbon atoms, Z represents oxygen and $R_{18}$ represents hydrogen, wherein

$R_1$ represents hydrogen, halogen, nitro, $C_1$–$C_4$ haloalkyl, $C_1$–$C_4$ alkoxy, $C_1$–$C_4$ alkyl, $-S(O)_m-C_1-C_4$ alkyl, $-SO_2-N(CH_3)_2$, $-SO_2-OCH_2CF_3$ or $-CO-R_6$,

$R_2$ represents hydrogen, fluorine, chlorine, nitro, amino, trifluoromethyl, methyl, methoxy, ethoxy or $-S(O)_m-C_1-C_4$ alkyl,

$R_6$ represents hydrogen, methyl, $C_3$–$C_5$ alkenyloxy, $C_3$–$C_5$ alkynyl, $C_1$–$C_3$ alkylthio, dimethylamino, methylamino, amino, or $C_1$–$C_5$ alkoxy that is optionally substituted by from 1 to 3 halogen atoms or by $C_1$–$C_3$ alkoxy, and

m is 0, 1 or 2.

11. N-(2-Chlorophenylsulphonyl)-N′-(4-difluoromethoxy-6-methylpyrimidin-2-yl)-urea according to claim 1.

12. N-(2-Methoxycarbonylphenylsulphonyl)-N′-[4,6-bis-(difluoromethoxy)-pyrimidin-2-yl]-urea according to claim 1.

13. N-(2-Methoxycarbonylphenylsulphonyl)-N′-(4-difluoromethoxy-6-methylpyrimidin-2-yl)-urea according to claim 1.

14. N-(2-Methoxycarbonylphenylsulphonyl)-N′-(4-difluoromethoxy-6-methoxypyrimidin-2-yl)-urea
according to claim 1.

15. N-(2-Nitrophenylsulphonyl)-N'-(4-difluoromethoxy-6-methylpyrimidin-2-yl)-urea according to claim 1.

16. Aminopyrimidines of the formula Va

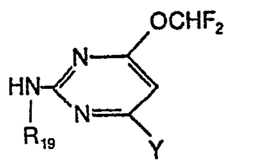

(Va)

in which Y is as defined for formula I in claim 1 and $R_{19}$ represents $C_1$–$C_3$ alkyl or $C_1$–$C_3$ alkoxy.

17. Process for the manufacture of compounds of the formula I according to claim 1, characterised in that an arylsulphonamide of the formula II

$$X{-}SO_2{-}NH_2 \quad (II),$$

in which X is as defined for formula I, is reacted in the presence of a base with an N-pyrimidinyl carbamate of the formula III

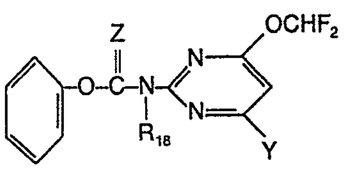

(III),

in which $R_{18}$, Y and Z are as defined for formula I, and, if desired, converted into the salts.

18. Process for the manufacture of compounds of the formula I according to claim 1, characterised in that an arylsulphonyl isocyanate or arylsulphonyl isothiocyanate of the formula IV

$$X{-}SO_2{-}N{=}C{=}Z \quad (IV),$$

in which X and Z are as defined for formula I, is reacted, if desired in the presence of a base, with an aminopyrimidine of the formula V

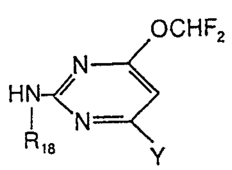

(V),

in which $R_{18}$ and Y are as defined for formula I, and, if desired, converted into the salts.

19. Process for the manufacture of compounds of the formula I according to claim 1 in which $R_{18}$ represents hydrogen, characterised in that an arylsulphonamide of the above formula II is reacted, optionally in the presence of a base, with an isocyanate or isothiocyanate of the formula VI

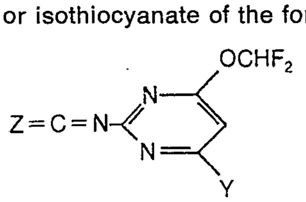

(VI),

in which Y and Z are as defined for formula I, and, if desired, converted into the salts.

20. Process for the manufacture of compounds of the formula 1 according to claim 1, characterised in that an N-arylsulphonyl carbamate of the formula VII

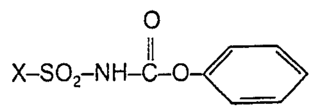

(VII),

in which X is as defined for formula I, is reacted with an aminopyrimidine of the above formula V and, if desired, converted into the salts.

21. Process for the manufacture of addition salts of the formula I according to any one of claims 17 to 20, characterised in that a sulphonylurea of the formula I is reacted with an amine, an alkali metal hydroxide or alkaline earth metal hydroxide, or with a quaternary ammonium base.

22. A herbicidal and plant growth-regulating agent, characterised in that it contains, in addition to carries and/or other adjuvants, at least one N-arylsulphonyl-N'-pyrimidinylurea of the formula I, claim 1, as active ingredient.

23. The use of N-arylsulphonyl-N'-pyrimidinylureas of the formula I, claim 1, or agents containing them, to control undesired plant growth.

24. The use of N-arylsulphonyl-N'-pyrimidinylureas of the formula I, claim 1, or of agents containing them, to regulate plant growth.

25. The use of N-phenylsulphonyl-N'-triazinyl- or N-phenylsulphonyl-N'-pyrimidinyl-ureas of the formula I, claim 1, or of agents containing them, to regulate the growth of cultivated plants in order to increase their yield.

26. The use according to claim 23 for the selective pre- or post-emergence control of weeds in useful plant crops.

27. The use according to claim 26 in sugar cane, maize and cotton crops.

28. The use according to claim 26 in soya crops.

29. The use according to claim 26 for controlling perennial weeds in useful plant crops.

30. The use according to claim 24 for inhibiting plant growth beyond the 2-leaf stage, characterised in that the active ingredients are applied pre-emergence.

31. The use according to claim 25 in soya crops.

32. The use according to claim 24 in cover crop leguminosae.

**Claims for the contracting state AT.**

1. A herbicidal and plant growth-regulating agent, characterised in that it contains, in addition to carriers and/or other adjuvants, at least one N-arylsulphonyl-N'-pyrimidinylurea of the formula I

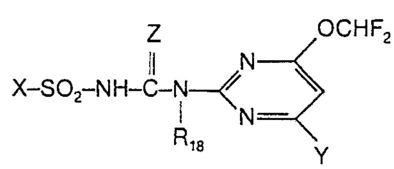

(I)

as active ingredient, wherein

X represents a radical of the formula

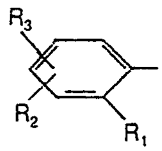

or

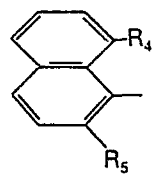

,

Y represents $C_1$–$C_3$ alkyl, $C_1$–$C_3$ haloalkyl, $C_1$–$C_3$ alkoxy, $C_1$–$C_3$ haloalkoxy, $C_2$–$C_3$ alkoxyalkyl, $C_1$–$C_3$ alkylthio, halogen or $-NR_{16}R_{17}$,

Z represents oxygen or sulphur,

$R_1$ represents hydrogen, halogen, cyano, nitro, $C_1$–$C_4$ haloalkyl, $C_1$–$C_4$ alkyl, $C_1$–$C_4$ alkoxy, $-CO-R_6$, $-NR_7R_8$, $-S(O)_m-C_1-C_4$ alkyl or $-SO_2R_9$,

$R_2$ represents hydrogen, fluorine, chlorine, bromine, nitro, trifluoromethyl, $-NR_{20}R_{21}$, methyl, ethyl, methoxy, ethoxy or $-S(O)_m-C_1-C_4$ alkyl,

$R_3$ represents hydrogen, fluorine, chlorine, bromine, amino, nitro or methoxy,

$R_6$ represents hydrogen, $C_1$–$C_4$ alkyl, $C_3$–$C_5$ alkenyloxy, $C_3$–$C_5$ alkynyloxy, $C_1$–$C_4$ haloalkyl, $C_1$–$C_5$ alkylthio, phenoxy, benzyloxy, $-NR_{10}R_{11}$, or $C_1$–$C_5$ alkoxy that is optionally substituted by from 1 to 3 halogen atoms or by $C_1$–$C_3$ alkoxy,

$R_7$ represents hydrogen, methoxy, ethoxy, $C_1$–$C_4$ alkyl or $-CO-R_{12}$,

$R_8$ represents hydrogen or $C_1$–$C_4$ alkyl,

$R_9$ represents an $O-R_{13}$ or $-NR_{14}R_{15}$ group,

$R_{13}$ represents $C_1$–$C_4$ alkyl that is optionally substituted by from 1 to 3 halogen atoms, or represents phenyl or benzyl,

$R_{18}$ represents hydrogen, $C_1$–$C_3$ alkyl or $C_1$–$C_3$ alkoxy, and

m is 0, 1 or 2,

and $R_4$ has the same meaning as $R_2$; $R_5$ has the same meaning as $R_1$; each of $R_{10}$, $R_{11}$, $R_{14}$ and $R_{20}$ has the same meaning as $R_7$; and each of $R_{12}$, $R_{15}$, $R_{16}$, $R_{17}$ and $R_{21}$ has the same meaning as $R_8$, and the salts of those compounds.

2. Agent according to claim 1, characterised in that X represents the optionally substituted phenyl radical.

3. Agent according to claim 1, characterised in that Y represents a radical containing a maximum of 2 carbon atoms.

4. Agent according to claim 1, characterised in that Z represents oxygen.

5. Agent according to claim 1, characterised in that $R_3$ and $R_4$ represent hydrogen.

6. Agent according to claim 1, characterised in that $R_{18}$ represents hydrogen or methyl.

7. Agent according to claim 1, characterised in that X represents the optionally substituted phenyl radical, Y represents a radical containing a maximum of 2 carbon atoms, Z represents oxygen and $R_3$ and $R_{18}$ represent hydrogen.

8. Agent according to claim 7, characterised in that $R_2$ represents hydrogen.

9. Agent according to claim 1, characterised in that X represents the optionally substituted phenyl nucleus, Y represents a radical containing a maximum of 2 carbon atoms, Z represents oxygen and $R_{18}$ represents hydrogen, wherein

$R_1$ represents hydrogen, halogen, nitro, $C_1$–$C_4$ haloalkyl, $C_1$–$C_4$ alkoxy, $C_1$–$C_4$ alkyl, $-S(O)_m-C_1-C_4$ alkyl, $-SO_2-N(CH_3)_2$, $-SO_2-OCH_2CF_3$ or $-CO-R_6$,

$R_2$ represents hydrogen, fluorine, chlorine, nitro, amino, trifluoromethyl, methyl, methoxy, ethoxy or $-S(O)_m-C_1-C_4$ alkyl,

$R_6$ represents hydrogen, methyl, $C_3$–$C_5$ alkenyloxy, $C_3$–$C_5$ alkynyl, $C_1$–$C_3$ alkylthio, dimethylamino, methylamino, amino, or $C_1$–$C_5$ alkoxy that is optionally substituted by from 1 to 3 halogen atoms or by $C_1$–$C_3$ alkoxy, and

m is 0, 1 or 2.

10. N-(2-Chlorophenylsulphonyl)-N′-(4-difluoromethoxy-6-methylpyrimidin-2-yl)-urea according to claim 1.

11. N-(2-Methoxycarbonylphenylsulphonyl)-N′-[4,6-bis-(difluoromethoxy)-pyrimidin-2-yl]-urea according to claim 1.

12. N-(2-Methoxycarbonylphenylsulphonyl)-N′-(4-difluoromethoxy-6-methylpyrimidin-2-yl)-urea according to claim 1.

13. N-(2-Methoxycarbonylphenylsulphonyl)-N′-(4-difluoromethoxy-6-methoxypyrimidin-2-yl)-urea according to claim 1.

14. N-(2-Nitrophenylsulphonyl)-N′-(4-difluoromethoxy-6-methylpyrimidin-2-yl)-urea according to claim 1.

15. Process for the manufacture of compounds of the formula I according to claim 1, characterised in that, either,

a) an arylsulphonamide of the formula II

$$X-SO_2-NH_2 \qquad (II),$$

in which X is as defined for formula I, is reacted in the presence of a base with an N-pyrimidinyl carbamate of the formula III

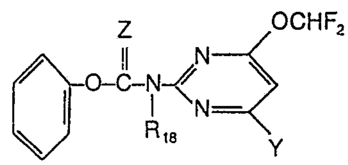

(III),

in which $R_{18}$, Y and Z are as defined for formula I, or

b) an arylsulphonyl isocyanate or arylsulphonyl isothiocyanate of the formula IV

$$X-SO_2-N=C=Z \qquad (IV),$$

in which X and Z are as defined for formula I, is reacted, if desired in the presence of a base, with an aminopyrimidine of the formula V

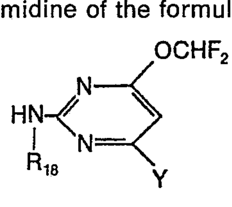

(V),

in which $R_{18}$ and Y are as defined for formula II, or

c) an N-arylsulphonyl carbamate of the formula VII

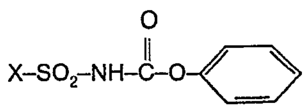

(VII),

in which X is as defined for formula I, is reacted with an aminopyrimidine of the above formula V and, if desired, there is a conversion into the salts by reacting a sulphonylurea of the formula I with an amine, an alkali metal hydroxide or alkaline earth metal hydroxide, or with a quaternary ammonium base.

16. Process for the manufacture of compounds of the formula I according to claim 1 in which $R_{18}$ represents hydrogen, characterised in that an arylsulphonamide of the above formula II is reacted, optionally in the presence of a base, with an isocyanate or isothiocyanate of the formula VI

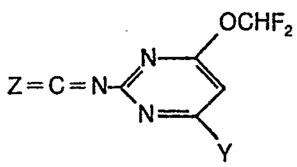

(VI),

in which Y and Z are as defined for formula I, and, if desired, converted into the salts.

17. The use of N-arylsulphonyl-N′-pyrimidinyl-ureas of the formula I, claim 1, or agents containing them, to control undesired plant growth.

18. The use of N-arylsulphonyl-N′-pyrimidinyl-ureas of the formula I, claim 1, or of agents containing them, to regulate plant growth.

19. The use of N-phenylsulphonyl-N′-triazinyl- or N-phenylsulphonyl-N′-pyrimidinyl-ureas of the formula I, claim 1, or of agents containing them, to regulate the growth of cultivated plants in order to increase their yield.

20. The use according to claim 17 or for the selective pre- or post-emergence control of weeds in useful plant crops.

21. The use according to claim 20 in sugar cane, maize and cotton crops.

22. The use according to claim 20 in soya crops.

23. The use according to claim 20 for controlling perennial weeds in useful plant crops.

24. The use according to claim 18 for inhibiting plant growth beyond the 2-leaf stage, characterised in that the active ingredients are applied pre-emergence.

25. The use according to claim 19 in soya crops.

26. The use according to claim 18 in cover crop leguminosae.

**Revendications pour les Etats contractants: BE, CH, DE, FR, GB, IT, LI, NL, SE**

1. N-arylsulfonyl-N′-pyrimidinylurées selon l'invention correspondant à la formule générale I.

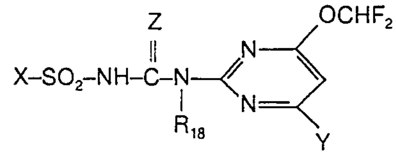

(I)

où

X représente un radical de formule

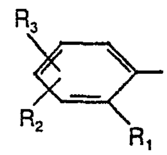

ou

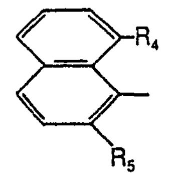

,

Y représente un alcoyle en $C_1$–$C_3$, halogénalcoyle en $C_1$–$C_3$, alkoxy en $C_1$–$C_3$, halogènalkoxy en $C_1$–$C_3$, alkoxyalcoyle en $C_2$–$C_3$, alcoylthio en $C_1$–$C_3$, halogène ou $-NR_{16}R_{17}$,

Z représente un oxygène ou un soufre,

$R_1$ représente un hydrogène, halogène, cyano, nitro, halogenalcoyle en $C_1$–$C_4$, alcoyle en $C_1$–$C_4$, alkoxy en $C_1$–$C_4$, $-CO-R_6$, $-S(O)_m$ alcoyle en $C_1$–$C_4$ ou $-SO_2R_9$,

$R_2$ représente un hydrogène, fluor, chlore, brome, nitro, trifluorométhyle, $-NR_{20}R_{21}$, méthyle, éthyle, méthoxy, éthoxy ou $-S(O)_m$ alcoyle en $C_1$–$C_4$,

$R_3$ représente un hydrogène, fluor, chlore, brome, amino, nitro ou méthoxy,

$R_6$ représente un hydrogène, alcoyle en $C_1$–$C_4$, alcényloxy en $C_3$–$C_5$, alcynyloxy en $C_3$–$C_5$, halogènalcoyle en $C_1$–$C_4$, alcoylthio en $C_1$–$C_5$, phénoxy, benzyloxy, $-NR_{10}R_{11}$ ou alkoxy en $C_1$–$C_5$ éventuellement substitué par de 1 à 3 atomes d'halogène ou un alkoxy en $C_1$–$C_3$,

$R_7$ représente un hydrogéne, méthoxy, éthoxy, alcoyle en $C_1$–$C_4$ ou $-CO-R_{12}$,

$R_8$ représente un hydrogène, alcoyle en $C_1$–$C_4$,

$R_9$ représente un groupe $-O-R_{13}$ ou $-NR_{14}R_{15}$,

$R_{13}$ représente un alcoyle en $C_1$–$C_4$, phényle ou benzyle éventuellement substitué par de 1 à 3 atomes d'halogène,

$R_{18}$ représente un hydrogène, alcoyle en $C_1$–$C_3$ ou alkoxy en $C_1$–$C_3$ et

m représente les nombres zéro, un ou deux,

où $R_4$ a la même signification que $R_2$ et $R_5$ la même signification que $R_1$; $R_{10}$, $R_{11}$, $R_{14}$ et $R_{20}$ la même signification que $R_7$; et $R_{12}$, $R_{15}$, $R_{16}$, $R_{17}$ et $R_{21}$ la même signification que $R_8$, ainsi que les sels de ses composés.

2. N-arylsulfonyl-N′-pyriminyl-urées de formule générale Ia

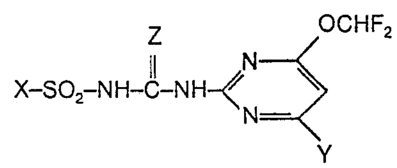

(Ia)

où X représente un radical de formule

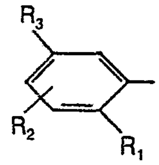 oder 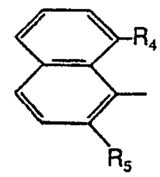 ,

Y représente un méthyl, éthyl, méthoxy, éthoxy, difluorométhoxy, diméthylamino ou éthylméthylamino,

Z représente un oxygène ou un soufre,

$R_1$ représente un hydrogène, fluor, chlore, brome, iode, cyano, nitro, trifluorométhyl, alcoyle en $C_1$–$C_4$, alkoxy en $C_1$–$C_4$, –CO–$R_6$, –$NR_7R_8$, –$S(O)_m$-alcoyle en $C_1$–$C_4$ ou –$SO_2$–$R_9$,

$R_2$ représente un hydrogène, fluor, chlore, brome, nitro, trifluorométhyl, amino, méthyl, éthyl, méthoxy, éthoxy ou –$S(O)_m$ alcoyle en $C_1$–$C_3$,

$R_3$ représente un hydrogène, fluor, chlore, brome, nitro ou méthoxy,

$R_6$ représente un hydrogène, alcoyle en $C_1$–$C_3$, alcényl oxy en $C_3$–$C_5$, alcynyloxy en $C_3$–$C_5$, alcoyl thio en $C_3$–$C_5$, phénoxy, benzyloxy, –$NR_{10}R_{11}$ ou alkoxy en $C_1$–$C_5$ éventuellement substitué par de 1 à 3 atomes d'halogène,

$R_7$ représente un hydrogène, méthoxy, éthoxy, alcoyle en $C_1$–$C_4$ ou –CO–$R_{12}$,

$R_8$ représente un hydrogène ou un alcoyle en $C_1$–$C_4$,

$R_9$ représente un groupe –O–$R_{13}$ ou –$NR_{14}R_{15}$,

$R_{13}$ représente un alcoyle en $C_1$–$C_4$, phényle ou benzyle et

m représente les nombres zéro, un ou deux, où $R_4$ a la même signification que $R_2$; $R_5$ a la même signification que $R_1$; $R_{10}$ et $R_{14}$ ont la même signification que $R_7$ et $R_{11}$, $R_{12}$ et $R_{15}$ la même signification que $R_8$, ainsi que les sels de ses composés.

3. Composés selon la revendication 1, caractérisés en ce que X représente le radical phénylène éventuellement substitué.

4. Composés selon la revendication 1, caractérisés en ce que Y représente un radical ayant au plus deux atomes de carbone.

5. Composés selon la revendication 1, caractérisés en ce que Z représente un oxygène.

6. Composés selon la revendication 1, caractérisés en ce que $R_3$ et $R_4$ représentent un hydrogène.

7. Composés selon la revendication 1, caractérisés en ce que $R_{18}$ représente un hydrogène ou un méthyle.

8. Composés selon la revendication 1, caractérisés en ce que X représente le radical phényle éventuellement substitué, Y un radical ayant au plus deux atomes de carbone, Z un oxygène et $R_3$ et $R_{18}$ un hydrogène.

9. Composés selon la revendication 8, caractérisés en ce que $R_2$ représente un hydrogène.

10. Composés selon la revendication 1, caractérisés en ce que X représente un noyau phényle éventuellement substitué, Y un radical ayant au plus deux atomes de carbone, Z un oxygène et $R_{18}$ un hydrogène, où $R_1$ représente un hydrogène, halogène, nitro, halogènalcoyle en $C_1$–$C_4$, alkoxy en $C_1$–$C_4$, alcoyle en $C_1$–$C_4$, –$S(O)_m$-alcoyle en $C_1$–$C_4$, –$SO_2$–$N(CH_3)_2$, –$SO_2$–$OCH_2CF_3$ ou –CO–$R_6$,

$R_2$ représente un hydrogène, fluor, chlore, nitro, amino, trifluorométhyl, méthyl, méthoxy, éthoxy ou –$S(O)_m$-alcoyle en $C_1$–$C_4$,

$R_6$ représente un hydrogène, méthyl, alcényloxy en $C_3$–$C_5$, alcynyloxy en $C_3$–$C_5$, alcoylthio en $C_1$–$C_3$, diméthylamino, méthylamino, amino ou alkoxy en $C_1$–$C_5$ éventuellement substitué par de 1 à 3 atomes d'halogène ou un alkoxy en $C_1$–$C_3$ et

m représente les nombres zéro, un ou deux.

11. N-(2-chlorophényl-sulfonyl)-N'-(4-difluorométhoxy-6-méthyl-pyrimidin-2-yl)-urée, selon la revendication 1.

12. N-(2-méthoxycarbonylphényl-sulfonyl)-N'-(4-difluorométhoxy-6-méthoxy-pyrimidin-2-yl)-urée, selon la revendicationn 1.

13. N-(2-nitrophényl-sulfonyl)-N'-(4-difluorométhoxy-6-méthyl-pyrimidin-2-yl)-urée, selon la revendication 1.

14. N-(2-méthoxycarbonylphényl-sulfonyl)-N'-[4,6-bis-difluorométhoxy)-pyrimidin-2-yl]-urée, selon la revendication 1.

15. N-(2-nitrophényl-sulfonyl)-N'-(4-difluorométhoxy-6-méthyl-pyrimidin-2-yl)-urée, selon la revendication 1.

16. Aminopyrimidines de formule Va

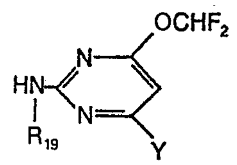 (Va)

où Y a la signification donnée sous la formule I dans la revendication 1 et $R_{19}$ représente un alcoyle en $C_1$–$C_3$ ou un alkoxy en $C_1$–$C_3$.

17. Procédé de préparation des composés de formule I selon la revendication 1, caractérisé en ce qu'on fait réagir un arylsulfonamide de formule II

$$X–SO_2–NH_2 \quad (II),$$

où X a la signification donnée sous la formule I, en présence d'une base avec un N-pyrimidinylcarbamate de formule III

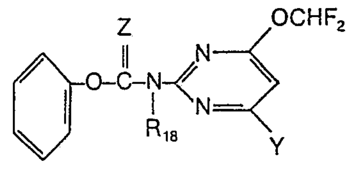 (III),

où $R_{18}$, Y et Z ont la signification donnée sous la formule I, et éventuellement en ce qu'on le transforme en un sel.

18. Procédé de préparation des composés de formule I, selon la revendication 1, caractérisé en ce qu'on fait réagir un arylsulfonylisocyanate ou -isothiocyanate de formule IV

$$X–SO_2–N=C=Z \quad (IV),$$

où X et Z ont les significations données sous la formule I, éventuellement en présence d'une base, avec une aminopyrimidine de formule V

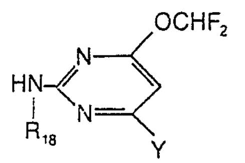

(V),

où $R_{18}$ et Y ont la signification donnée sous la forme I, et éventuellement en ce qu'on le transforme en les sels.

19. Procédé de préparation des composés de formule I selon la revendication 1, où $R_{18}$ représente un hydrogène, caractérisé en ce qu'on fait réagir un arylsulfonamide de formule II indiquée ci-dessus, éventuellement en présence d'une base avec un isocyanate ou isothiocyanate de formule VI

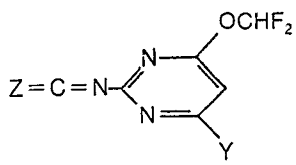

(VI),

où Y et Z ont la signification donnée sous la formule I, et éventuellement en ce que le transforme en les sels.

20. Procédé de préparation des composés de formule I selon la revendication 1, caractérisé en ce qu'on fait réagir un N-arylsulfonylcarbamate de formule VII

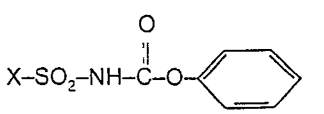

(VII),

où X a la signification donnée sous la formule I, avec une aminopyrimidine de formule V indiquée ci-dessus et éventuellement en ce qu'on le transforme en les sels.

21. Procédé de préparation de sels d'addition de formule I selon l'une des revendications 17 à 20, caractérisé en ce qu'on fait réagir une sulfonylurée de formule I avec une amine, un hydroxyde de métal alcalin ou de métal alcalino-terreux ou une base d'ammonium quaternaire.

22. Herbicide et agent de régulation de la croissance des plantes caractérisé en ce qu'il contient outre des supports et/ou autres additifs, comme matière active au moins une N-arylsulfonyl-N'-pyrimidinyl-urée de formule I de la revendication 1.

23. Application des N-arylsulfonyl-N'-pyrimidinyl-urée de formule I de la revendication 1 ou des agents qui les contiennent à la lutte contre la croissance végétale indésirable.

24. Application des N-arylsulfonyl-N'-pyrimidinyl-urée de formule I de la revendication 1 ou des agents qui les contiennent à la régulation de la croissance des plantes.

25. Application de N-méthylsulfonyl-N'-triazinyl ou -pyrimidinyl-urée de formule I de la revendication 1 ou des agents qui les contiennent à la régulation de la croissance des plantes cultivées dans un but d'accroissement du rendement.

26. Application selon la revendication 23 à la lutte sélective en pré ou en post-levée contre les mauvaises herbes dans les cultures de plantes utiles.

27. Application selon la revendication 26 dans les cultures de canne à sucre, de maïs, ou de coton.

28. Application selon la revendication 26 dans les cultures de•soja.

29. Application selon la revendication 26 à la lutte contre les mauvaises herbes pérennes dans les cultures de plantes utiles.

30. Application selon la revendication 24, à la suppression de la croissance végétale au-delà du stade de feuilles, caractérisée en ce qu'on applique les matières actives en pré-levée.

31. Application selon la revendication 25 dans les cultures de soja.

32. Application selon la revendication 24 dans les légumineuses protectrices du sol.

**Revendications pour l'Etat contractant: AT**

1. Agent herbicide et de régulation de la croissance, caractérisé en ce qu'il contient outre des supports et/ou autres additifs, comme matière active, au moins une N-arylsulfonyl-N'-pyrimidinyl-urée de formule I

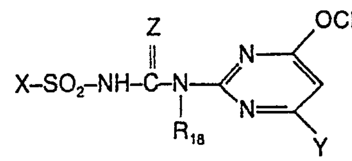

(I)

où

X représente un radical de formule

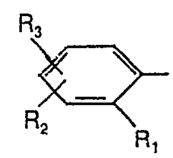

ou

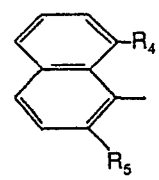

,

X représente un alxoyle en $C_1$–$C_3$, halogenalcoyle en $C_1$–$C_3$, alkoxy en $C_1$–$C_3$, halogènalkoxy en $C_1$–$C_3$, alkoxyalxoyle en $C_2$–$C_3$, alcoylthio en $C_1$–$C_3$, halogène ou $-NR_{16}R_{17}$,

Z représente un oxygène ou un soufre,

$R_1$ représente un hydrogène, halogène, cyano, nitro, halogenalcoyle en $C_1$–$C_4$, alcoyle en $C_1$–$C_4$, alkoxy en $C_1$–$C_4$, $-CO-R_6$, $-S(O)_m$ alcoyle en $C_1$–$C_4$ ou $-SO_2R_9$,

$R_2$ représente un hydrogène, fluor, chlore, brome, nitro, trifluorométhyle, $-NR_{20}R_{21}$, méthyle, éthyle, méthoxy, éthoxy ou $-S(O)_m$ alcoyle en $C_1$–$C_4$,

$R_3$ représente un hydrogène, fluor, chlore, brome, amino, nitro ou méthoxy,

$R_6$ représente un hydrogène, alcoyle en $C_1$–$C_4$, alcényloxy en $C_3$–$C_5$, alcynyloxy en $C_3$–$C_5$, halogènalcoyle en $C_1$–$C_4$, alcoylthio en $C_1$–$C_5$, phénoxy

benzyloxyn $-NR_{10}R_{11}$ ou alkoxy en $C_1$–$C_5$ éventuellement substitué par de 1 à 3 atomes d'halogène ou un alkoxy en $C_1$–$C_3$,

$R_7$ représente un hydrogène, méthoxy, éthoxy, alcoyle en $C_1$–$C_4$ ou $-CO-R_{12}$,

$R_8$ représente un hydrogène, alcoyle en $C_1$–$C_4$,

$R_9$ représente un groupe $-O-R_{13}$ ou $-NR_{14}R_{15}$,

$R_{13}$ représente un alcoyle en $C_1$–$C_4$, phényle ou benzyle éventuellement substitué par de 1 à 3 atomes d'halogène,

$R_{18}$ représente un hydrogène, alcoyle en $C_1$–$C_3$ ou alkoxy en $C_1$–$C_3$ et

m représente les nombres zéro, un ou deux,

où $R_4$ a la même signification que $R_2$ et $R_5$ la même signification que $R_1$; $R_{10}$, $R_{11}$, $R_{14}$ et $R_{20}$ la même signification que $R_7$; et $R_{12}$, $R_{15}$, $R_{16}$, $R_{17}$ et $R_{21}$ la même signification que $R_8$, ainsi que les sels de ces composés.

2. Agent selon la revendication 1, caractérisé en ce que X représente le radical phénylène éventuellement substitué.

3. Agent selon la revendication 1, caractérisé en ce que Y représente un radical ayant au plus deux atomes de carbone.

4. Agent selon la revendication 1, caractérisé en ce que Z représente un oxygène.

5. Agent selon la revendication 1, caractérisé en ce que $R_3$ et $R_4$ représentent un hydrogène.

6. Agent selon la revendication 1, caractérisé en ce que $R_8$ représente un hydrogène ou un méthyle.

7. Agent selon la revendication 1, caractérisé en ce que X représente le radical phényle éventuellement substitué, Y un radical ayant au plus deux atomes de carbone, Z un oxygène et $R_3$ et $R_{18}$ un hydrogène.

8. Agent selon la revendication 7, caractérisé en ce que $R_2$ représente un hydrogène.

9. Agent selon la revendication 1, caractérisé en ce que X représente un noyau phényle éventuellement substitué, Y un radical ayant au plus deux atomes de carbone, Z un oxygène et $R_{18}$ un hydrogène, où

$R_1$ représente un hydrogène, halogène, nitro, halogènalcoyle en $C_1$–$C_4$, alkoxy en $C_1$–$C_4$, alcoyle en $C_1$–$C_4$, $-S(O)_m$-alcoyle en $C_1$–$C_4$, $-SO_2-N(CH_3)_2$, $-SO_2-OCH_2CF_3$ ou $-CO-R_6$,

$R_2$ représente un hydrogène, fluor, chlore, nitro, amino, trifluorométhyl, méthyl, méthoxy, éthoxy ou $-S(O)_m$-alcoyle en $C_1$–$C_4$,

$R_6$ représente un hydrogène, méthyl, alcényloxy en $C_3$–$C_5$, alcynyloxy en $C_3$–$C_5$, alcoylthio en $C_1$–$C_3$, diméthylamino, méthylamino, amino ou alkoxy en $C_1$–$C_5$ éventuellement substitué par de 1 à 3 atomes d'halogène ou un alkoxy en $C_1$–$C_3$ et

m représente les nombres zéro, un ou deux.

10. N-(2-chlorophényl-sulfonyl)-N'-(4-difluorométhoxy-6-méthyl-pyrimidin-2-yl)-urée selon la revendication 1.

11. N-(2-méthoxycarbonylphényl-sulfonyl)-N'-(4-difluorométhoxy-6-méthoxy-pyrimidin-2-yl)-urée selon la revendication 1.

12. N-(2-méthoxycarbonylphényl-sulfonyl)-N'-(4-difluorométhoxy-6-méthyl-pyrimidin-2-yl)-urée selon la revendication 1.

13. N-(2-méthoxycarbonylphényl-sulfonyl)-N'-[4,6-bis-difluorométhoxy)-pyrimidin-2-yl]-urée selon la revendication 1.

14. N-(2-nitrophényl-sulfonyl)-N'-(4-difluorométhoxy-6-méthyl-pyrimidin-2-yl)-urée selon la revendication 1.

15. Procédé de préparation des composés de formule I selon la revendication 1, caractérisé en ce que ou bien

(a) on fait réagir un arylsulfonamide de formule II

$$X-SO_2-NH_2 \qquad (II),$$

où X a la signification donnée sous la formule I, en présence d'une base avec un N-pyrimidinylcarbamate de formule III

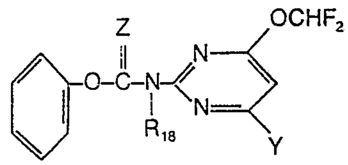
(III),

où $R_{18}$, Y et Z ont la signification donnée sous la formule I, ou bien

(b) on fait réagir un arylsulfonylisocyanate ou -isothiocyanate de formule IV

$$X-SO_2-N=C=Z \qquad (IV),$$

où X et Z ont les significations données sous la formule I, éventuellement en présence d'une base, avec une aminopyrimidine de formule V

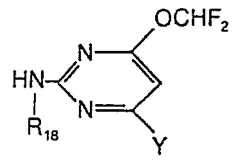
(V),

où $R_{18}$ et Y ont la signification donnée sous la formule I, ou bien

(c) on fait réagir un N-arylsulfonylcarbamate de formule VII

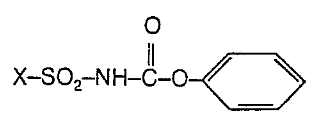
(VII),

où X a la signification donnée sous la formule I, avec une aminopyrimidine de formule V indiquée ci-dessus et éventuellement en ce qu'on le transforme en les sels de manière à faire réagir une sulfonylurée de formule I avec une amine, un hydroxyde de métal alcalin ou de métal alcalino-terreux ou une base d'ammonium quaternaire.

16. Procédé de préparation des composés de formule I selon la revendication 1, où $R_{18}$ représente un hydrogène, caractérisé en ce qu'on fait réagir un arylsulfonamide de formule II indiquée ci-dessus, éventuellement en présence d'une

base avec un isocyanate ou isothiocyanate de formule VI

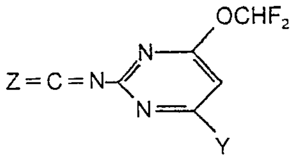

(VI),

où Y et Z ont la signification donnée sous la formule I, et éventuellement en ce qu'on le transforme en les sels.

17. Application des N-arylsulfonyl-N'-pyrimidinyl-urées de formule I de la revendication 1 ou des agents qui contiennent à la lutte contre la croissance végétale indésirable.

18. Application des N-arylsulfonyl-N'-pyrimidinyl-urées de formule I de la revendication 1 ou des agents qui les contiennent à la régulation de la croissance des plantes.

19. Application de N-méthylsulfonyl-N'-triazinyl ou -pyrimidinyl-urées de formule I de la revendication 1 ou des agents qui les contiennent à la régulation de la croissance des plantes cultivées dans un but d'accroissement du rendement.

20. Application selon la revendication 17 à la lutte sélective en pré ou en post-levée contre les mauvaises herbes dans les cultures de plantes utiles.

21. Application selon la revendication 20 dans les cultures de canne à sucre, de maïs, ou de coton.

22. Application selon la revendication 20 dans les cultures de soja.

23. Application selon la revendication 20 à la lutte contre les mauvaises herbes pérennes dans les cultures de plantes utiles.

24. Application selon la revendication 18, à la suppression de la croissance végétale au-delà du stade de feuilles, caractérisée en ce qu'on applique les matières actives en pré-levée.

25. Application selon la revendication 19 dans les cultures de soja.

26. Application selon la revendication 18 dans les légumineuses protectrices du sol.